# EUROPEAN PATENT APPLICATION

(11) **EP 3 205 662 A2**
(43) Date of publication of application: **16.08.2017**
(21) Application number: 17162645.0
(22) Date of filing: 10.11.2011
(51) Int. Cl.: C07K 14/16, G06F 19/14, A61P 31/18

(54) **HIGHLY IMMUNOGENIC HIV P24 SEQUENCES**

(30) Priority: 10.11.2010 US 412060 P
(62) Divisional of application: 11784463.9
(71) Applicant: Laboratorios del Dr. Esteve S.A., 08041 Barcelona (ES); Fundació Privada Institut de Recerca de la SIDA-Caixa, 08916 Badalona (ES); Institució Catalana de Recerca i Estudis Avançats, 08010 Barcelona (ES); University of Washington, Seattle, WA 98105-4608 (US)
(72) Inventor: MOTHE PUJADAS, Beatriz, E-08015 Barcelona (ES); BRANDER, Christian, E-09016 Tiana (ES); MULLINS, James I., Seattle, WA 98105 (US)
(74) Representative: ABG Patentes, S.L.

(57) **Abstract**

The invention relates to peptides comprising part or all of a conserved element within a Center-of Tree (COT) sequence derived from a family of polypeptides encoded by naturally occurring variants of HIV. The invention also relates to immunogenic compositions and vaccines comprising said peptides. The invention also relates to methods for the identification of HIV controller patients based on the detection of the T cells of the patient to mount a cytotoxic T cell response against said peptides and to methods for the identification of immunogenic peptides within a family of variant polypeptides.

## Description

### FIELD OF THE INVENTION

The invention relates to immunogenic compositions and methods for immunogen design based on the use of peptides derived from conserved elements within sequences of antigenic proteins and, more in particular, antigenic ancestor p24 sequences from HIV.

### BACKGROUND OF THE INVENTION

HIV-1 vaccine design will have to contend with global HIV-1 sequence diversity. Focusing on highly conserved regions of the HIV-1 genome for vaccine immunogen designs could help to overcome this problem considerably. In addition, mounting immune responses against highly conserved segments of the genome could also prevent the rapid emergence of CTL escape variants as such mutations will likely cause significant reductions in viral replicative fitness. Furthermore, aiming vaccine-induced responses to most conserved targets would avoid inducing responses to highly mutable epitopes that serve as immunodominant decoys that actively defer responses away from protective, invariant epitopes.

Studies with diverse cohorts of clade B and clade C-infected individuals have shown that cytotoxic T-cell responses against HIV-1 Gag are correlated with relative control of HIV-1 *in vivo. See* Kiepiela P, et al., Nat. Med. 2007; 13:46-53; Masemola A, et al., J. Virol. 2004; 78:3233-3243; Mothe B, et al., Dis. Markers 2009; 27:105-120; and Zuñiga R, et al., J. Virol. 2006; 80:3122-3125. Several hypotheses have been put forward to explain these observations. It has been proposed that the rapid representation of epitopes derived from the Gag proteins contained in the infecting virus particles may render the Gag-specific CTL response more effective than other responses. *See* Sacha J, et al., J. Immunol. 2007; 178:2746-2754. Alternatively, the high level of conservation of the gag sequence may also reflect the need for a maintained structural integrity of the Gag protein and its low tolerance for escape mutations. *See* Schneidewind A, et al., J. Virol. 2008; 82:5594-5605. It has also been argued that HIV-1 Gag may contain a high number of CTL epitopes restricted by HLA alleles associated with relative HIV-1 control (such as HLA-B27, B57 and others), although data from large cohort-based studies suggest this is quite unlikely. *See* Borghans J, et al., PLoS ONE 2007; 2:e920 and Kiepiela, 2007, *supra.* A specific accumulation of epitopes in Gag presented to these beneficial HLA alleles is also not supported by the described high degree of HLA promiscuity among well-defined CTL epitopes that allow these "good" epitopes to be presented in the context of other HLA alleles. In addition, in all studies reporting the beneficial effects of Gag-specific responses, some HIV-1-infected non-controllers mount detectable responses against Gag as well, raising the question of why these individuals are not able to control viral replication. This has even been reported for Gag-specific responses restricted by the protective HLA-B57 allele in subjects infected by HIV-1 strains with intact wildtype sequences containing the dominant and supposedly protective epitope who nevertheless do not control HIV replication. *See* Migueles S., et al., J. Virol. 2003; 77:6889-6898. These studies, together with previously published data on the impact of subdominant CTL responses in HIV-1 as well as in SIV infections, suggest that functional characteristics, including functional avidity and variant cross-reactivity may be determinant parameters to mount beneficial, protective CTL responses. *See* Frahm N., et al., Nat. Immunol. 2006; 7:173-178 and Friedrich T, et al., J. Virol. 2007; 81:3465-3476.

Also, our understanding of the host immune response to any portion of the viral proteome may be incomplete due to technical limitations and the use of antigenic test reagents may also introduce specific bias. Although earlier studies using overlapping peptide (OLP) sets of variable length (15-20 amino acids in length) did not produce significantly different response rates, our own observations in HIV-1 infected individuals using 18mer OLP or optimally defined CTL epitopes, indicated that short 10mer peptides would provide a much more realistic view of the total responses than longer OLP. *See* Draenert R, et al., J. Immunol. Methods 2003; 275:19-29 and Frahm, 2007, *supra.* Shorter peptides might require less antigen processing before being effectively presented by HLA class I molecules and may thus identify more responses and responses of lower functional avidity than the corresponding 18mer OLP sets generally used in *ex-vivo* analyses.

### DESCRIPTION OF THE FIGURES

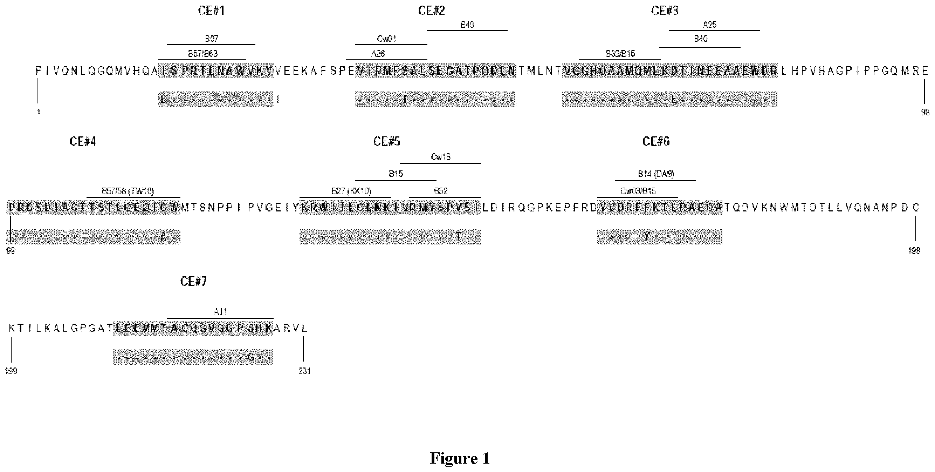

**Figure 1****. COT-M Gag-p24 sequence and location of CE segments.** The location and HLA restriction elements of known optimally-defined CTL epitopes are indicated above the protein sequence of COT-M Gag p24 described previously. *See* Rolland M, et al., PLOS Pathogen. 2007; 3:e157. Shaded boxes indicate the 7 CE segments located within p24 with alternative residues included beneath.

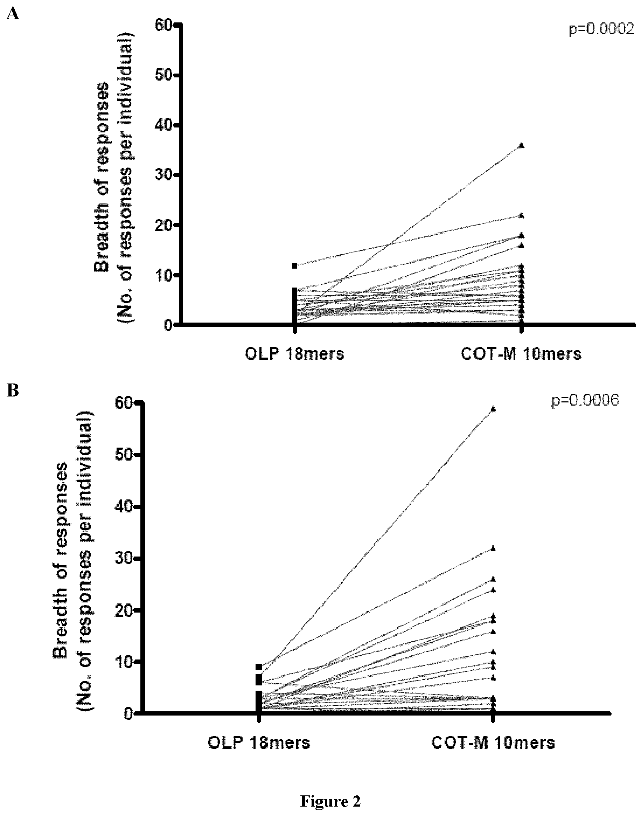

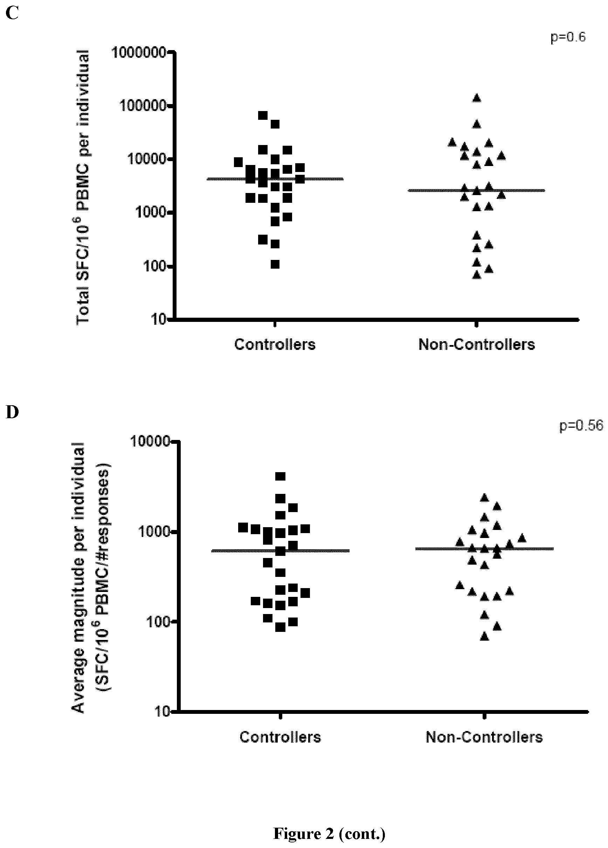

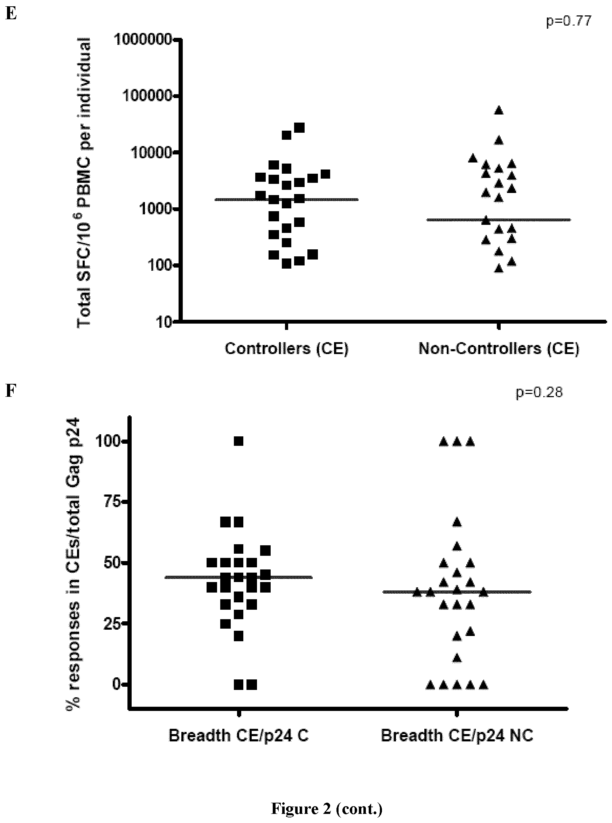

**Figure 2****. Increased sensitivity of detection of responses to Gag p24 with the COT-M 10mer set of peptides.** IFN-γ ELISpot responses against Gag p24 elicited either by consensus B overlapping 18mer or COT-M 10mer peptide sets in 25 HIV-1 controllers **(A)** and 25 HIV-1 non-controllers **(B)** P-values indicated reflect the level of significance of the increase in breath of responses when using 10mer peptide sets (two-tailed Wilcoxon matched paired test) compared to 18mers. Total magnitude of responses **(C)** and average magnitude of responses **(D)** to COT-M Gag p24 10mer peptides is shown for 25 controllers and 25 non-controllers. **(E)** Total magnitude of responses confined to CE regions only in controllers vs. non-controllers. **(F)** Relative dominance of CE specific responses compared to the rest of Gag p24 in controllers vs. non-controllers. Lines represent median values and indicated p values for C, D, E and F are based on comparisons using Mann-Whitney t-test.

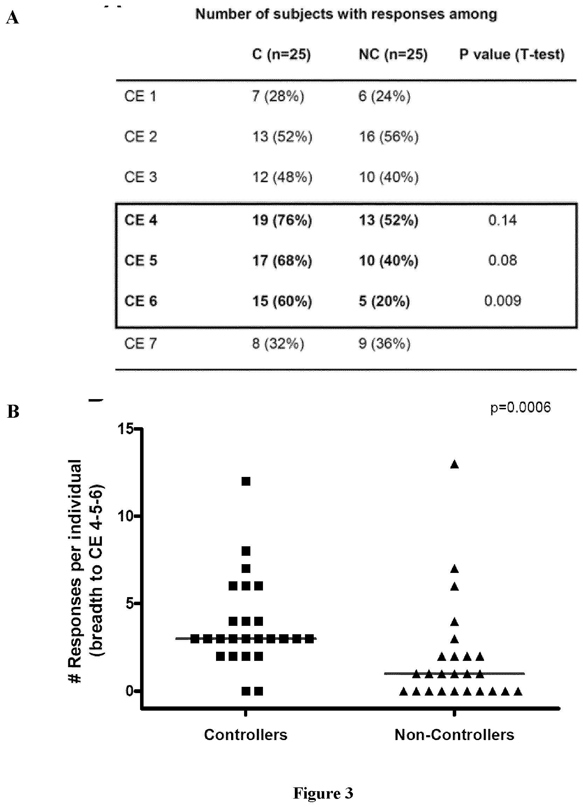

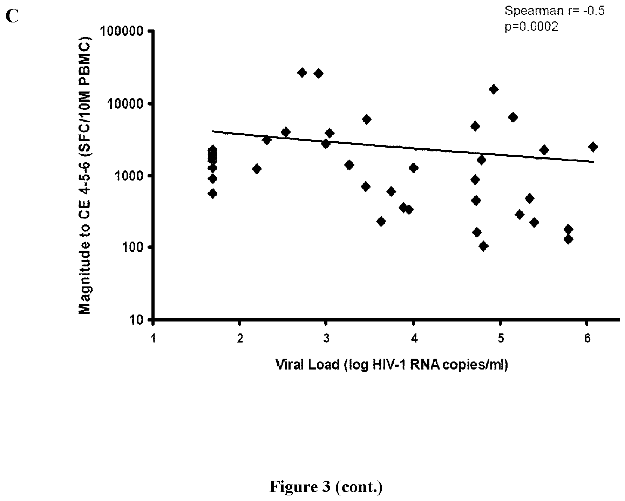

**Figure 3****. CE containing HLA-B14, -B27 and B57 restricted, protective CTL epitopes are predominantly targeted by HIV-1 controllers. (A)** The frequency of recognition of the 7 different CE is shown for 25 HIV-1 controllers (C) and 25 non-controllers (NC), respectively. Recognition of a CE was considered positive when at least one 10mer peptide entirely contained within the CE sequence was targeted. CE regions targeted by at least 50% more controllers than non-controllers are boxed and p-values indicated (T test). **(B)** Breath of response to the combination of CE 4+5+6 regions in controllers versus non-controllers is shown. Horizontal lines represent median values and Mann-Whitney t-test p value is shown. **(C)** Correlation between the cumulative magnitude of IFN-gamma ELISpot responses to CE 4+5+6 and HIV-1 viral loads in all 50 tested individuals is shown (p-value is based on Spearman's rank test).

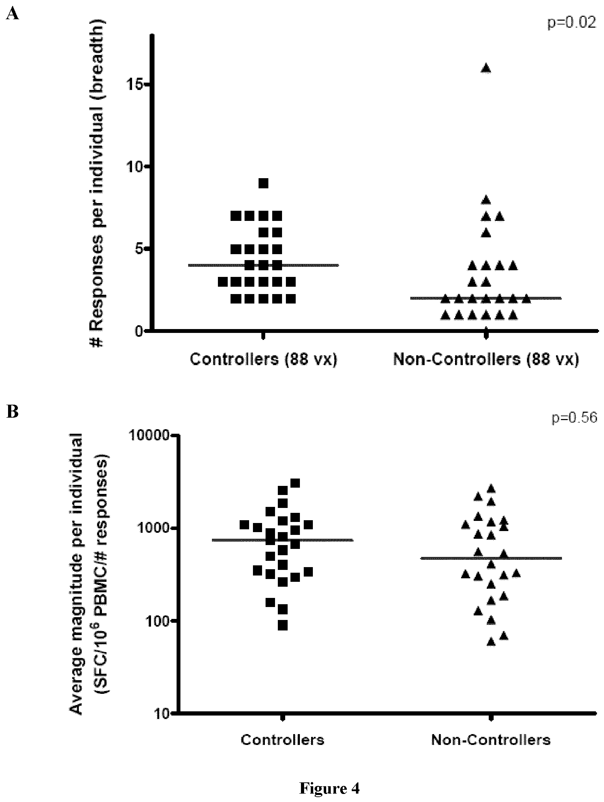

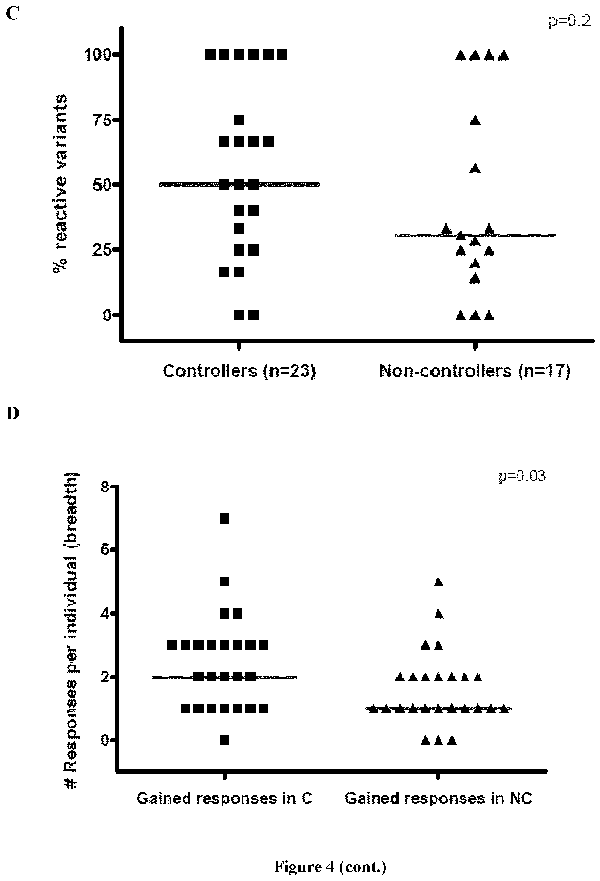

**Figure 4****. Higher variant recognition in HIV-1 controllers. (A)** The number of individual responses to the COT-M Gag p24 variant peptides (n=88) and **(B)** the average magnitude per individual response is compared between the 25 HIV-1 controllers and 25 non-controllers. Responses to two or three adjacent 10mer peptides were considered as one single response. **(C)** Percentage of variant peptides that were reactive when the COT-M sequence elicited a response ("cross-reactive responses") and **(D)** responses to variant peptides for which the COT-M sequence did not elicit a response ("gained responses") are shown. Horizontal lines represent median values and Mann-Whitney t-test p values are indicated.

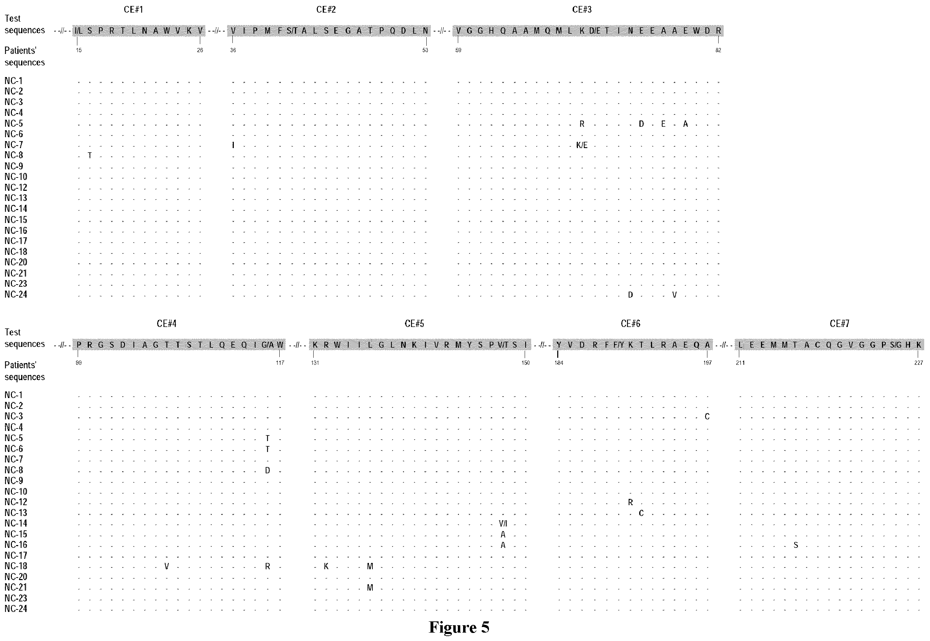

**Figure 5****. Autologous Gag p24 sequence alignment in 21 HIV-1 non-controllers.** Shaded boxes indicate the 7 CE sequences located within p24 with variant residues included. Alignment of autologous Gag p24 bulk (consensus) sequences obtained from 21 HIV-1-non-controllers and amino acid polymorphisms identified across all 7 segments are shown.

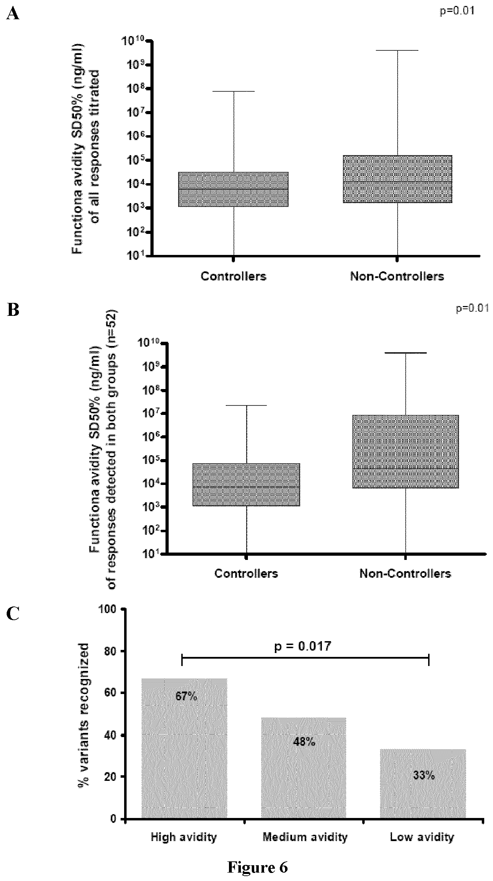

**Figure 6****. High avidity responses are enriched in HIV-1 controllers and mediate superior variant recognition compared to low-avidity responses. (A)** Comparison of functional avidity of all COT-M Gag p24 responses titrated in controllers (n= 219 individual responses) versus non-controllers (n=255 individual responses). Median values, interquartile range and p value (Mann-Whitney t test) are shown. **(B)** Comparison of functional avidity limited to responses targeting the same 10mer OLP in controllers and non-controllers (n=52 responses, two-tailed Wilcoxon matched paired test). **(C)** Association between functional avidity and cross-reactivity is shown. Responses with functional avidities in the first quartile of all titrated responses (SD50% < 1,401ng/ml), the second and third quartile (SD50% 1,401-71,594ng/ml) or the fourth quartile (SD50% > 71,594ng/ml) were defined as "high", "intermediate" and low" avidity responses, respectively. The percentage of variants that elicited a response was compared between the three groups (Fisher Exact Test).

### DESCRIPTION OF THE INVENTION

The present invention relates to a prototype vaccine immunogen, experimentally validated, which includes the most conserved regions of HIV-1 p24, referred to as "conserved elements" (CE). The immune reactivity against these regions and against the rest of HIV-1 Gag p24 or of the remainder of the proteome in HIV-1 has been compared in individuals that control HIV well ("HIV-controllers") and subjects that poorly control their viral replication ("HIV non-controllers"). The design of the CE sequences was based on the identification of regions in the HIV-1 proteome with typically at least 98% sequence conservation across all independent group M sequences at the Los Alamos HIV database. *See* http://www.hiv.lanl.gov/content/index, September 2011. For HIV-1 Gag p24, studied as a proof-of-concept protein, this produced 7 segments, ranging from 12 to 24 amino acids in length, corresponding to a total of 124 residues of p24. A side-by-side comparison of 10mer versus 18mer peptides for Gag p24 was conducted, revealing 2-3 times higher response rates when using the shorter peptide test sets.

The results show that when using sensitive peptide test sets, the same overall breadth and magnitude of the CTL responses to Gag p24 can be detected equally by HIV-1 controllers and non-controllers. However, high avidity and cross-reactive responses against Gag p24 are significantly enriched in HIV-1 controllers. These data suggest that HIV-1 non-controllers are unable to induce or maintain responses of desirable functionality to these targets. The results also present functional confirmation of the potential usefulness of this novel CE-based HIV-1 vaccine immunogen approach that can readily be expanded to the rest of the viral genome and which could provide immunity against heterologous viral challenge.

### A. Immunogenic peptides of the invention

Thus, in a first aspect, the invention relates to a peptide having the capacity to induce HIV-1-specific cytotoxic T lymphocytes (CTL), the sequence of which comprises part or all of a conserved element within a Center-of-Tree (COT) sequence derived from a family of polypeptides encoded by naturally occurring variants of HIV-1.

The term "peptide", as used herein, refers to a sequence of amino acids, analogues or mimetics having substantially similar or identical functionality. The term "peptide" also includes analogues having synthetic and natural amino acids joined together by peptide bonds.

A cytotoxic T lymphocyte assay can be used to monitor the cellular immune response following subgenomic immunization with a viral sequence against homologous and heterologous HIV strains. *See* Burke S, et al., J. Inf. Dis. 1994; 170:1110-1119 and Tigges M, et al., J. Immunol, 1996; 156:3901-3910. Conventional assays utilized to detect T cell responses include, for instance, proliferation assays, lymphokine secretion assays, direct cytotoxicity assays and limiting dilution assays. For example, antigen-presenting cells that have been incubated with a peptide can be assayed for their ability to induce CTL responses in responder cell populations. Antigen-presenting cells can be cells such as peripheral blood mononuclear cells (PBMC) or dendritic cells (DC). Alternatively, mutant non-human mammalian cell lines that are deficient in their ability to load MHC class I molecules with internally processed peptides and that have been transfected with the appropriate human MHC class I gene, can be used to test the capacity of a peptide of interest to induce *in vitro* primary CTL responses.

PBMC can be used as the responder cell source of CTL precursors. The appropriate antigen-presenting cells are incubated with the peptide after which the protein-loaded antigen-presenting cells are incubated with the responder cell population under optimized culture conditions. Positive CTL activation can be determined by assaying the culture for the presence of CTL that kill radiolabeled target cells, both specific peptide-pulsed targets as well as target cells expressing endogenously processed forms of the antigen from which the peptide sequence was derived. For example, the target cells can be radiolabeled with ⁵¹Cr and cytotoxic activity can be calculated from radioactivity released from the target cells. Another suitable method allows the direct quantification of antigen-specific T cells by staining with Fluorescein-labeled HLA tetrameric complexes. *See* Altman J, et al., Proc. Natl. Acad. Sci. USA 1993; 90:10330-10334 and Altman J, et al., Science 1996; 274:94-96. Other relatively recent technical developments include staining for intracellular lymphokines and interferon release assays or ELISpot assays.

The term "conserved element", as used herein, refers to a region of a sequence which is substantially constant among all related variant sequences when said sequences are compared. For sequence comparison, typically one sequence acts as a reference sequence to which test sequences are compared. When using a sequence comparison algorithm, test and reference sequences are typically input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

In a preferred embodiment, the sequence is considered as being a conserved element if it shows at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 995 or 100% identity with respect to reference sequence.

Optimal alignment of sequences for comparison can be conducted, for instance, by the Smith-Waterman local homology algorithm, by the Needleman-Wunsch homology alignment algorithm, by the Pearson-Lipman similarity search method, by computerized implementations of these algorithms or by manual alignment and visual inspection. *See* Smith T, Waterman M, Adv. Appl. Math. 1981; 2:482-489; Needleman S, Wunsch C, J. Mol. Biol. 1970; 48:443-453; Pearson W, Lipman D, Proc. Natl. Acad. Sci. USA 1988; 85:2444-2448; the GAP, BESTFIT, FASTA and TFASTA programs, Wisconsin Genetics Software Package, Genetics Computer Group, Madison, WI, USA; Ausubel F, et al., Eds., "Short Protocols in Molecular Biology", 5th Ed. (John Wiley and Sons, Inc., New York, NY, USA, 2002).

One example of a useful algorithm is PILEUP. This program creates a multiple sequence alignment from a group of related sequences using progressive, pairwise alignments to show relationship and percent sequence identity. It also plots a tree or dendogram showing the clustering relationships used to create the alignment. *See* Feng D, Doolittle R, J. Mol. Evol. 1987; 35:351-360. The method is similar to the CLUSTAL algorithm. *See* Higgins D, Sharp P, Gene 1998; 73:237-244 and CABIOS 1989; 5:151-153. The program can align up to 300 sequences, each of a maximum length of 5,000 nucleotides or amino acids. The multiple alignment procedure begins with the pairwise alignment of the two most similar sequences, producing a cluster of two aligned sequences. This cluster is then aligned to the next most related sequence or cluster of aligned sequences. Two clusters of sequences are aligned by a simple extension of the pairwise alignment of two individual sequences. The final alignment is achieved by a series of progressive, pairwise alignments. The program is run by designating specific sequences and their amino acid or nucleotide coordinates for regions of sequence comparison and by designating the program parameters. For example, a reference sequence can be compared to other test sequences to determine the percent sequence identity relationship using the following parameters: default gap weight (3.00), default gap length weight (0.10), and weighted end gaps.

Other examples of algorithms suitable for determining percent sequence identity and sequence similarity are BLAST and BLAST 2.0 algorithms. *See* Altschul S, et al., Nuc. Acids Res. 1977; 25:3389-3402 and Altschul S, et al., J. Mol. Biol. 1990; 215:403-410. The BLAST and BLAST 2.0 programs are used, with the parameters described herein, to determine percent sequence identity for the nucleic acids and proteins of the invention. Software for performing BLAST analyses is publicly available through the National Center for Biotechnology Information. *See* http://blast.ncbi.nlm.nih.gov/blast.cgi, September 2011. This algorithm involves first identifying high scoring sequence pairs (HSPs) through the recognition of short words of length W in the query sequence, which either match or satisfy some positive-valued threshold score T when aligned with a word of the same length in a database sequence. T is referred to as the neighborhood word score threshold. *See* Altschul, 1997, 1990, *supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) of 10, M=5, N=4 and a comparison of both strands. For amino acid sequences, the BLASTP program uses as defaults a word length of 3, and expectation (E) of 10, and the BLOSUM62 scoring matrix alignments (B) of 50, expectation (E) of 10, M=5, N4, and a comparison of both strands. *See* Henikoff S, Henikoff J, Proc. Natl. Acad. Sci. USA 1989; 89:10915-10919. The BLAST algorithm also performs a statistical analysis of the similarity between two sequences. *See* Karlin S, Altschul S, Proc. Natl. Acad. Sci. USA 1993; 90:5873-5787. One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01.

The term "ancestral sequence", as used herein, refers to a sequence that is at the base node of the maximum likelihood phylogeny of a family of variant sequences. The ancestral sequence is thus approximately equidistant from the different sequences.

The term "Center-of-Tree sequence" or "COT", as used herein, refers to a sequence from which the average evolutionary distance to each tip of a phylogenetic diagram of related variant sequences has been minimized. *See* Nickle D, et al., Science 2003; 299, 1515-1517.

Typically, the family of variant polypeptides correspond to polypeptides encoded by the genomes of viruses that exist as highly diverse viral populations. However, some highly diverse viruses such as FIV, HIV-1, HIV-2, hepatitis C and endogenous retroviruses like PERV, do not appear to evolve across their host populations through a succession of variants, where one prototypical strain is replaced by successive uniform strains. Instead, an evolutionary tree of viral sequences can form a "star-burst pattern", with most of the variants approximately equidistant from the center of the star-burst. This star-burst pattern indicates that multiple, diverse circulating strains evolve from a common ancestor. Computational methods can be used to determine ancestral sequences for highly diverse viruses, such as, for example, FIV, HIV-1, HIV-2, hepatitis C and endogenous retroviruses.

Methods for the determination of the COT sequence from a family of sequences are described in the art. *See* Rolland M, et al., J. Virol. 2007; 81:8507-8514, Mullins J, et al., US 7,655,744 and WO2005/001029. Typically, the determination of the COT or ancestral sequences is carried out by computational methods from the nucleic acid sequences of circulating viruses based on the principle of maximum likelihood. In a preferred embodiment, the sequences are nucleic acid sequences of circulating viruses. The sequences of the viruses in the samples typically share a common feature, such as being from the same viral strain, subtype or group. A phylogeny is constructed by using a model of evolution that specifies the probabilities of nucleotide substitutions in the replicating viral nucleic acids. At positions in the sequences where the nucleotides differ (i.e. at the site of a mutation), the methodology assigns one of the nucleotides to the node (i.e. the branch point of the lineages) such that the probability of obtaining the observed viral sequences is maximized. The assignment of nucleotides to the nodes is based on the predicted phylogeny or phylogenies. For each data set, several sequences from a different viral strain, subtype or group are used as an outgroup to root the sequences of interest. A model of sequence substitutions and then a maximum likelihood phylogeny are determined for each data set (e.g. subtype and outgroup). The maximum likelihood phylogeny is the one that has the highest probability of giving the observed nucleic acid sequences in the samples. The sequence at the base node of the maximum likelihood phylogeny is referred to as the ancestral sequence or most recent common ancestor.

Alternatively, a second method is to choose, for a given nucleotide site and a given node on the tree, the nucleotide that maximizes the probability of obtaining the observed sequences of circulating viruses, allowing for all possible assignments of nucleotides at the other nodes on the tree. This second method maximizes the marginal likelihood of a particular assignment. For these methods, the reconstruction of the ancestral sequence (i.e. ancestral state) need not result in only a single determined sequence, however. It is possible to choose a number of ancestral sequences, ranked in order of their likelihood.

Alternatively, in the case of HIV populations, a second layer of modeling can be added to the maximum likelihood phylogenetic analysis, in particular the layer is added to the model of evolution that is employed in the analysis. This second layer is based on coalescent likelihood analysis. The coalescent is a mathematical description of a genealogy of sequences, taking account of the processes that act on the population. If these processes are known with some certainty, the use of the coalescent can be used to assign prior probabilities to each type of tree. Taken together with the likelihood of the tree, the posterior probability can be determined that a determined phylogenetic tree is correct given the data. Once a tree is chosen, the ancestral states are determined, as described above. Thus, coalescent likelihood analysis can also be applied to determine the sequence of an ancestral viral sequence (e.g. a founder, or Most Recent Common Ancestor (MRCA), sequence).

In a typical embodiment, maximum likelihood phylogeny analysis is applied to determine a COT or an ancestor sequence (e.g. an ancestral viral sequence). Typically, between 20 and 1000 nucleic acid sequence samples are used that have a common feature, such as a viral strain, subtype or group (e.g. samples encompassing a worldwide diversity of the same subtype). Additional sequences from other viruses (e.g. another strain, subtype, or group) are obtained and used as an outgroup to root the viral sequences being analyzed. The samples of viral sequences are determined from presently circulating or endogenous viruses, identified from the database (e.g. GenBank and Los Alamos HIV sequence databases), or from similar sources of sequence information. The sequences are aligned using the CLUSTALW algorithm. *See* Thompson J., et al., Nucleic Acids Res. 1994, 22:4673-4680. These alignments are refined using GDE. *See* Smith J, et al., CABIOS 1994; 10:671-675. The amino acid sequences are also translated from the nucleic acid sequences. Gaps are manipulated so that they are inserted between codons.

The sequence of the peptides of the invention comprises part or all of the conserved elements within the COT or, in order to increase sequence diversity coverage, may further include second most frequent variants for each conserved element. The inclusion of such frequent "toggled" amino acids increases coverage at those sites among all group M sequences to >99%.

In a preferred embodiment, the sequence of the peptides of the invention have a length of 10 amino acids of less, including 9 amino acids, 8 amino acids, 7 amino acids, 6 amino acids, 5 amino acids or less.

The term "polypeptide", as used herein, refers to a polymer in which the monomers are amino acids and are joined together through peptide or disulphide bonds. The term includes post-translational modifications of the polypeptide, for example, glycosylations, acetylations, and phosphorylations.

The acronym "HIV" is used herein to refer to human immunodeficiency viruses generically and includes HIV type 1 (HIV-1), HIV type 2 (HIV-2) or other HIV viruses, including, for example, the HIV-1, HIV-2, emerging HIV and other HIV subtypes and HIV variants, such as widely dispersed or geographically isolated variants. For example, an ancestral viral gene sequence can be determined for the *env* and *gag* genes of HIV-1, such as for HIV-1 subtypes A, B, C, D, E, F, G, H, J, and K, and intersubtype recombinants such as AG, AGI, and for groups M, N, O or for HIV-2 viruses or HIV-2 subtypes A or B. In specific embodiments, ancestral viral sequences are determined for the *env* genes of HIV-1 subtypes B and/or C, or for the *gag* genes of subtypes B and/or C. In other embodiments, the ancestral viral sequence is determined for other HIV genes or polypeptides, such as *pol* or the auxiliary genes or polypeptides.

In a preferred embodiment, the HIV is a group M HIV. Group M is the predominant circulating HIV-1 group. It has been divided into subtypes, denoted with letters, and sub-subtypes, denoted with numerals. Subtypes A1, A2, A3, A4, B, C, D, E, F1, F2, G, H, J, and K are currently recognized. HIV-1 subtypes, also called clades, are phylogenetically linked strains of HIV-1 that are approximately the same genetic distance from one another; in some cases, subtypes are also linked geographically or epidemiologically. Genetic variation within a subtype can be 15 to 20 percent or more, whereas variation between subtypes or divergent members of the same subtype is usually 25 to 35 percent. Over the past decade, advances in full-genome sequencing of HIV have led to the identification of circulating and unique recombinant forms (CRFs and URFs, respectively). These are the result of recombination between subtypes within a dually infected person, from whom the recombinant forms are then passed to other people. The recombinant progeny are classified as circulating recombinant forms if they are identified in three or more people with no direct epidemiologic linkage; otherwise they are described as unique recombinant forms.

The term "naturally occurring variants", as used herein, refers to nucleic acid sequences of a selected HIV-1 or HIV-2 gene which can be used for the identification of the ancestral or COT sequences may derive from presently and/or formerly circulating viruses and can be identified from existing databases (e.g. GenBank and Los Alamos sequence databases). The sequence of circulating viruses can also be determined by molecular biology methodologies. *See* Brown T, "Gene Cloning" (Chapman & Hall, London, England, 1995); Watson R, et al., "Recombinant DNA", 2nd Ed. (Scientific American Books, New York, NY, USA, 1992); Alberts B, et al., "Molecular Biology of the Cell" (Garland Publishing Inc., New York, NY, USA, 2008); Innis M, et al., Eds., "PCR Protocols. A Guide to Methods and Applications" (Academic Press Inc., San Diego, CA, USA, 1990); Erlich H, Ed., "PCR Technology. Principles and Applications for DNA Amplification" (Stockton Press, New York, NY, USA, 1989); Sambrook J, et al., "Molecular Cloning. A Laboratory Manual" (Cold Spring Harbor Laboratory Press, Cold Spring Harbor, NY, USA, 1989); Bishop T, et al., "Nucleic Acid and Protein Sequence. A Practical Approach" (IRL Press, Oxford, England, 1987); Reznikoff W, Ed., "Maximizing Gene Expression" (Butterworths Publishers, Stoneham, MA, USA, 1987); Davis L, et al., "Basic Methods in Molecular Biology" (Elsevier Science Publishing Co., New York, NY, USA, 1986), Schleef M, Ed., "Plasmid for Therapy and Vaccination" (Wiley-VCH Verlag GmbH, Weinheim, Germany 2001).

The term "HIV gene" refers to any sequence within the HIV genome that contains information necessary for expression of a polypeptide or protein and may include non-translated sequences. The term is also intended to include any combination of gene(s), gene fragment(s), non-transcribed sequence(s) or non-translated sequence(s) that are present on the same DNA molecule. Thus, the term "gene", as sometimes used generically, can also include nucleic acid molecules comprising cDNA and cDNA clones.

Non-limiting examples of HIV proteins that may be suitable for use in the embodiments presented herein include the HIV Gag proteins p53, p24, p17, p7, p6, p2 or p1, the HIV Env glycoproteins gp120, gp41 or gp160, HIV enzymes including integrase (p31), reverse transcriptase (p51 or p66), RNase H (p15), protease (p10), the HIV Nef proteins (p25/p27), the HIV Vif protein p23, the HIV Rev protein p19, the HIV Vpr protein (p12/p10), HIV Vpu protein (p16) or HIV Tat proteins (p16/p14).

In a preferred embodiment, the HIV gene is the *gag* gene. The term "*gag* gene", as used herein, refers to a gene that codes for the Gag polyprotein, which is processed during maturation to MA (matrix protein, p17); CA (capsid protein, p24); SP1 (spacer peptide 1, p2); NC (nucleocapsid protein, p7); SP2 (spacer peptide 2, p1) and p6. Sequences of *gag* genes found in HIV isolates can be readily found (e.g. GenBank and Los Alamos HIV sequence databases).

In a still more preferred embodiment, a polypeptide encoded by the *gag* gene is p24. As used herein, the term "p24" refers to the gene product of the *gag* region of HIV, characterized as having an apparent relative molecular weight of about 24,000 daltons designated p24 and which corresponds to the capsid protein of the HIV virion. The term "p24" also refers to modifications and fragments of p24 having the immunological activity of p24.

In a preferred embodiment, the sequences of conserved elements within p24 sequences are selected from the group consisting of the sequences as defined in Table 1.

**Table 1: Sequences of conserved elements within HIV p24.**

| Sequence | SEQ ID NO: |
|---|---|
| ISPRTLNAWVKV | 1 |
| LSPRTLNAWVKV | 2 |
| VIPMFSALSEGATPDQDLN | 3 |
| VIPMFTALSEGATPDQDLN | 4 |
| VGGHQAAMQMLKDTINEEAAEWDR | 5 |
| VGGHQAAMQMLKETINEEAAEWDR | 6 |
| PRGSDIAGTTSTLQEQIGW | 7 |
| PRGSDIAGTTSTLQEQIAW | 8 |
| KRWIILGLNKIVRMYSPVSI | 9 |
| KRWIILGLNKIVRMYSPTSI | 10 |
| YVDRFFKTLRAEQA | 11 |
| YVDRFYKTLRAEQA | 12 |
| LEEMMTACQGVGGPSHK | 13 |
| LEEMMTACQGVGGPGHK | 14 |

In a preferred embodiment, the peptide of the invention has a length of 10 amino acids or less. In a still more preferred embodiment, the peptide according to the present invention is selected from the group consisting of the peptides shown in Table 2.

**Table 2: Sequences of peptides derived from the conserved elements within HIV p24 tested for the T-cell response in HIV controllers vs. HIV non-controllers subjects.**

| Peptide | SEQ ID NO: | Peptide | SEQ ID NO: |
|---|---|---|---|
| SPRTLNAWV | 15 | TSTLQEQIAW | 26 |
| ISPRTLNAW | 16 | KRWIILGLNK | 27 |
| LSPRTLNAW | 17 | GLNKIVRMY | 28 |
| EVIPMFSAL | 18 | VRMYSPVSI | 29 |
| EVIPMFTAL | 19 | VRMYSPTSI | 30 |
| VIPMFTAL | 20 | RMYSPVSI | 31 |
| SEGATPDQDL | 21 | RMYSPTSI | 32 |
| GHQAAMQML | 22 | YVDRFFKTL | 33 |
| KDTINEEAA | 23 | YVDRFYKTL | 34 |
| KETINEEAA | 24 | DRFFKTLRA | 35 |
| DTINEEAAEW | 25 | DRFYKTLRA | 36 |

### B. Polynucleotides and vectors

In another aspect, the invention relates to a polynucleotide encoding a peptide according to the invention.

The term "polynucleotide", as used herein, refers to single-stranded or doublestranded polymers of nucleotide monomers (nucleic acids), including, but not limited to, 2'-deoxyribonucleotides (DNA) and ribonucleotides (RNA) linked by internucleotide phosphodiester bond linkages. It will be understood that the polynucleotides of the invention encode the peptides of the invention without substantially comprising additional regions of the HIV genome. Thus, in the case wherein the peptides of the invention derive from conserved elements within the p24 COT sequence, the polynucleotides of the invention contain those sequences encoded said conserved elements without including additional p24 sequences.

In another aspect, the invention relates to a vector comprising a polynucleotide as defined in the invention.

The term "vector" is used to denote a nucleic acid molecule, linear or circular, that comprises a segment encoding the peptide of interest operably linked to additional segments that provide for its autonomous replication in a host cell of interest. Preferably, the vector is an expression vector, which is defined as a vector, which in addition to the regions of the autonomous replication in a host cell, contains regions operably linked to the polynucleotide of the invention and which are capable of enhancing the expression of the peptide according to the invention.

Thus, suitable vectors according to the present invention include prokaryotic vectors, such as pUC18, pUC19, and Bluescript plasmids and derivatives thereof, like the mp18, mp19, pBR322, pMB9, ColE1, pCRl and RP4 plasmids; phages and shuttle vectors, such as pSA3 and pAT28 vectors; expression vectors in yeasts, such as 2-micron plasmid type vectors; integration plasmids; YEP vectors; centromeric plasmids and analogues; expression vectors in insect cells, such as the vectors of the pAC series and of the pVL series; expression vectors in plants, such as vectors of the pIBI, pEarleyGate, pAVA, pCAMBIA, pGSA, pGWB, pMDC, pMY, pORE series and analogues; and expression vectors in superior eukaryotic cells either based on viral vectors (e.g. adenoviruses, viruses associated to adenoviruses, retroviruses and lentiviruses) as well as non-viral vectors, such as the pSilencer 4.1-CMV (Ambion), pcDNA3, pcDNA3.1/hyg pHCMV/Zeo, pCR3.1, pEFl/His, pIND/GS, pRc/HCMV2, pSV40/Zeo2, pTRACER-HCMV, pUB6/V5-His, pVAXl, pZeoSV2, pCI, pSVL and pKSV-10, pBPV-1, pML2d and pTDTl vectors.

### C. Immunogenic compositions and therapeutic uses thereof

The results provided in the present invention identify promising immunogenic sequences for a broadly applicable HIV-1 vaccine that covers global HIV-1 diversity. Thus, in another aspect, the invention relates to an immunogenic composition or a vaccine comprising a peptide, a nucleic acid or a vector according to the invention.

The term "immunogenic composition" refers to a composition that elicits an immune response that produces antibodies or cell-mediated immune responses against a specific immunogen. Immunogenic compositions can be prepared, for instance, as injectables such as liquid solutions, suspensions, and emulsions. The term "antigenic composition" refers to a composition that can be recognized by a host immune system. For example, an antigenic composition contains epitopes that can be recognized by humoral and/or cellular components of a host immune system.

The term "vaccine" refers to an immunogenic composition for *in vivo* administration to a host, which may be a primate, especially a human host, to confer protection against a disease, particularly a viral disease.

In a further aspect, the invention relates to a peptide, a nucleic acid, a vector or an immunogenic composition according to the invention for use in medicine.

In a preferred embodiment, the invention relates to a pharmaceutical composition comprising a peptide, nucleic acid or vector according to the invention and a pharmaceutically acceptable carrier.

A "pharmaceutically acceptable carrier," "pharmaceutically acceptable diluent," or "pharmaceutically acceptable excipient", or "pharmaceutically acceptable vehicle," used interchangeably herein, refer to a non-toxic solid, semisolid or liquid filler, diluent, encapsulating material or formulation auxiliary of any conventional type. A pharmaceutically acceptable carrier is essentially non-toxic to recipients at the employed dosages and concentrations and is compatible with other ingredients of the formulation. For example, the carrier for a formulation containing polypeptides would not normally include oxidizing agents and other compounds that are known to be deleterious to polypeptides. Suitable carriers include, but are not limited to water, dextrose, glycerol, saline, ethanol, and combinations thereof. The carrier can contain additional agents such as wetting or emulsifying agents, pH buffering agents, or adjuvants that enhance the effectiveness of the formulation. Adjuvants could for example be selected from the group consisting of: AlK(SO4)2, AlNa(SO4)2, AlNH4 (SO4), silica, alum, Al(OH)3, Ca3(PO4)2, kaolin, carbon, aluminum hydroxide, muramyl dipeptides, N-acetyl-muramyl-L-threonyl-D-isoglutamine (thr-DMP), N-acetyl-nornuramyl-L-alanyl-D-isoglutamine (CGP 11687, also referred to as nor-MDP), N-acetylmuramyul-L-alanyl-D-isoglutaminyl-L-alanine-2-(1 '2'-dipalmitoyl-sn - glycero-3-hydroxphosphoryloxy)-ethylamine (CGP 19835A, also referred to as MTP-PE), RIBI (MPL+TDM+CWS) in a 2 percent squalene/TWEEN® 80 emulsion, lipopolysaccharides and its various derivatives, including lipid A, Freund's Complete Adjuvant (FCA), Freund's Incomplete Adjuvants, Merck Adjuvant 65, polynucleotides (e.g. poly IC and poly AU acids), wax D from *Mycobacterium tuberculosis,* substances found in *Corynebacterium parvum, Bordetella pertussis,* and members of the genus Brucella, Titermax, ISCOMS, Quil A, ALUN, Lipid A derivatives, choleratoxin derivatives, HSP derivatives, LPS derivatives, synthetic peptide matrixes or GMDP, interleukin 1, interleukin 2, Montanide ISA-51 and QS-21, CpG oligonucleotide, poly I:C, and IL-12, IL-15 and/or GM-CSF. *See* Hunter R, US 5,554,372 and Jager E, Knuth A, WO1997/28816.

In a further aspect, the invention relates to a peptide, a nucleic acid, a vector, an immunogenic composition or a vaccine according to the invention for use in the treatment or prevention of a disease resulting from HIV-infection. Alternatively, the invention relates to the use of a peptide, a nucleic acid, a vector, an immunogenic composition or a vaccine according to the invention for the manufacture of a medicament for the treatment or prevention of a disease resulting from HIV-infection. Alternatively, the invention relates to a method for the treatment or prevention in a subject of a disease resulting from HIV-infection that comprises the administration to said subject of a peptide, nucleic acid, vector, or immunogenic composition or a vaccine according to the invention.

The term "treat" or "treatment" is used to designate the administration of an immunogenic composition of the invention or of a medicament containing it to control the progression of the disease before or after clinical signs have appeared. Control of the disease progression is understood to mean the beneficial or desired clinical results that include, but are not limited to, reduction of the symptoms, reduction of the duration of the disease, stabilization of pathological states (specifically to avoid additional deterioration), delaying the progression of the disease, improving the pathological state and remission (both partial and total). The control of progression of the disease also involves an extension of survival, compared with the expected survival if treatment was not applied.

The expression "disease associated with a HIV infection" includes a state in which the subject has developed AIDS, but also includes a state in which the subject infected with HIV has not shown any sign or symptom of the disease. Thus, the immunogenic compositions of the invention when administered to a subject that has no clinical signs of the infection can have a preventive activity, since they can prevent the onset of the disease. The immunogenic compositions are capable of preventing or slowing the infection and destruction of healthy CD4+ T cells in such a subject. It also refers to the prevention and slowing the onset of symptoms of the acquired immunodeficiency disease such as extreme low CD4+ T cell count and repeated infections by opportunistic pathogens such as *Mycobacteria spp., Pneumocystis carinii,* and *Pneumocystis cryptococcus.* Beneficial or desired clinical results include, but are not limited to, an increase in absolute naive CD4+ T-cell count (range 10-3520), an increase in the percentage of CD4+ T-cell over total circulating immune cells (range 1-50 percent), and/or an increase in CD4+ T-cell count as a percentage of normal CD4+ T-cell count in an uninfected subject (range 1-161 percent). "Treatment" can also mean prolonging survival of the infected subject as compared to expected survival if the subject did not receive any HIV targeted treatment.

The beneficial prophylactic or therapeutic effect of an HIV immunogenic composition in relation to HIV infection or AIDS symptoms include, for example, preventing or delaying initial infection of an individual exposed to HIV; reducing viral burden in an individual infected with HIV; prolonging the asymptomatic phase of HIV infection; maintaining low viral loads in HIV infected patients whose virus levels have been lowered via anti-retroviral therapy (ART); increasing levels of CD4 T cells or lessening the decrease in CD4 T cells, both HIV-1 specific and non-specific, in drug naive patients and in patients treated with ART, increasing overall health or quality of life in an individual with AIDS; and prolonging life expectancy of an individual with AIDS. A clinician can compare the effect of immunization with the patient's condition prior to treatment, or with the expected condition of an untreated patient, to determine whether the treatment is effective in inhibiting AIDS.

As used herein, "AIDS" refers to the symptomatic phase of HIV infection, and includes both Acquired Immune Deficiency Syndrome (commonly known as AIDS) and "ARC," or AIDS-Related Complex, as described by Adler, Brit. Med. J. 294: 1145 (1987). The immunological and clinical manifestations of AIDS are well known in the art and include, for example, opportunistic infections and cancers resulting from immune deficiency.

In a preferred embodiment, the immunogenic compositions of the invention are preventive compositions. "Prevention" is understood to mean the administration of an immunogenic composition of the invention or of a medicament containing it in a initial or early stage of the infection, to avoid the appearance of clinical signs.

The immunogenic compositions of the invention may be useful for the therapy of HIV-1 infection. While all animals that can be afflicted with HIV-1 or their equivalents can be treated in this manner (e.g. chimpanzees, macaques, baboons or humans), the immunogenic compositions of the invention are directed particularly to their therapeutic uses in humans. Often, more than one administration may be required to bring about the desired therapeutic effect; the exact protocol (dosage and frequency) can be established by standard clinical procedures.

The present invention further relates to preventing or reducing symptoms associated with HIV infection. These include symptoms associated with the minor symptomatic phase of HIV infection, including, for example, shingles, skin rash and nail infections, mouth sores, recurrent nose and throat infection and weight loss. In addition, further symptoms associated with the major symptomatic phase of HIV infection, include, for instance, oral and vaginal thrush (Candida), persistent diarrhea, weight loss, persistent cough and reactivated tuberculosis or recurrent herpes infections, such as cold sores (herpes simplex). Other symptoms of full-blown AIDS which can be treated in accordance with the present invention include, for instance, diarrhoea, nausea and vomiting, thrush and mouth sores, persistent, recurrent vaginal infections and cervical cancer, persistent generalized lymphadenopathy (PGL), severe skin infections, warts and ringworm, respiratory infections, pneumonia, especially *Pneumocystis carinii pneumonia* (PCP), herpes zoster (or shingles), nervous system problems, such as pains, numbness or "pins and needles" in the hands and feet, neurological abnormalities, Kaposi's sarcoma, lymphoma, tuberculosis or other similar opportunistic infections.

Beneficial effects of the peptides, nucleic acids and vectors of the invention include, for example, preventing or delaying initial infection of an individual exposed to HIV, reducing viral burden in an individual infected with HIV, prolonging the asymptomatic phase of HIV infection, maintaining low viral loads in HIV infected patients whose virus levels have been lowered via anti-retroviral therapy (ART), increasing levels of CD4 T cells or lessening the decrease in CD4 T cells, both HIV-1 specific and non-specific, in drug naive patients and in patients treated with ART, increasing overall health or quality of life in an individual with AIDS and prolonging life expectancy of an individual with AIDS. A clinician can compare the effect of immunization with the patient's condition prior to treatment, or with the expected condition of an untreated patient, or in a clinical trial of individuals treated and untreated with the vaccine to determine whether the treatment is effective in inhibiting AIDS.

### D. Antibodies of the invention

In another aspect, the invention relates to an antibody or a polypeptide comprising an antigen-binding region thereof that binds specifically to a peptide according to the invention.

The term "antibody", as used herein, refers to a protein consisting of one or more proteins substantially encoded by all or part of the recognized immunoglobulin genes, including but not limited to polyclonal antibodies, monoclonal antibodies, and antigen-binding fragments thereof such as, for instance, F(ab')₂ and Fab fragments, and single chain antibodies. The term antibody includes any type of known antibody, such as, for example, polyclonal antibodies, monoclonal antibodies and genetically engineered antibodies, such as chimeric antibodies, humanized antibodies, primatized antibodies, human antibodies and bispecific antibodies.

"Chimeric antibodies" are understood as antibodies constructed with variable regions of an antibody of a species (usually a mammal in which the monoclonal antibody was generated) and constant regions of another species (that species in which the chimeric antibody is going to be used). The objective of said construct is to obtain an antibody with the original monoclonal antibody but which is less immunogenic and better tolerated in the subject who is going to be treated, with an improved serum half-life and which can be recognized by immunological effector mechanisms, i.e., the complement, the Fc receptor of cytotoxic cells or other specific immunoglobulin receptors which show species specificity. In a preferred embodiment, the chimeric antibodies are formed by murine variable regions and human constant regions.

"Humanized antibody" is understood as an antibody from a non-human organism, typically a murine antibody, which conserves the antigen binding properties of the parent antibody, but which is less immunogenic in human beings. This can be achieved by means of different processes, which include (a) grafting the complete nonhuman variable domains into human constant regions to generate chimeric antibodies; (b) grating only the nonhuman complementarity determining regions (CDR) in a human framework and the constant regions, with or without retaining the critical framework residues; and (c) transplanting the complete nonhuman variable domains, but "concealing them" with a section similar to the human variable domain by means of replacing the surface residues.

"Primatized antibody" is understood as a recombinant antibody that has been genetically manipulated to contain the heavy and light variable domains of a monkey antibody (or of another primate), particularly an antibody of a cynomolgus monkey, and containing sequences of a human constant domain, preferably the constant domain of human gamma 1 or 4 immunoglobulin (or a PE variant). The preparation of said antibodies is described in Newman et al., Biotechnology, 10: 1458-1460 (1992); and in patent documents US 5,658,570 and US 6,113,898. It has been described that these antibodies show a high degree of homology with human antibodies, i.e., 85-98%, they have human effector functions, they have lower immunogenicity and can show a high affinity for human antigens. Another very effective means for generating recombinant antibodies is described by Newman, Biotechnology, 10: 1455-1460 (1992).

"Human antibody" is understood as an antibody integrally containing human light and heavy chains as well as constant regions, produced by means of any of the known standard methods. A more extensive definition is found on the "Definitions" section.

The invention also comprises the use of fragments of the different types of antibodies mentioned above which substantially preserve the anti-angiogenic activity of the antibody. The term "antibody fragment" includes antibody fragments such as Fab, F(ab')2, Fab', single chain Fv fragments (scFv), diabodies and nanobodies.

Papain digestion of antibodies produces two identical antigen binding fragments referred to as "Fab" fragments, each with a single antigen binding site, and a residual "Fc" fragment, the name of which reflects its capacity for readily crystallizing. Pepsin treatment yields an F(ab')2 fragment which has two antigen binding sites and which is still capable of cross-linking to the antigen.

"Fv" is the minimal antibody fragment containing a complete antigen binding and antigen recognition site. This region consists of a variable domain of a variable light chain and heavy chain dimer in a strong noncovalent association. In this configuration the three hypervariable regions of each variable domain interact to define an antigen binding site on the surface of the VH-VL dimer. As a whole, the six hypervariable regions confer antigen-antibody specificity to the antibody. However, even a single variable domain (or half an Fv, which comprises only three hypervariable regions specific for an antigen) has antigen recognition and binding capacity, although with less affinity than the complete binding site.

The Fab fragment also contains the constant domain of the light chain and the first constant domain (CH1) of the heavy chain. Fab' fragments differ from Fab fragments in the addition of a few residues at the carboxy terminus of the domain CH1 of the heavy chain, including one or more cysteines of the antibody hinge region.

The "single chain Fv" or "scFv" antibody fragments comprise the VH and VL domains of an antibody, in which these domains are present in a single polypeptide chain. Preferably, the Fv polypeptide additionally comprises a linker polypeptide between the VH and VL domains which allows the scFv to form the desired structure for antigen binding. For a review of scFv, see Pluckthun in The Pharmacology of Monoclonal Antibodies, vol. 113, Rosenburg and Moore eds., Springer-Verlag, N.Y., pp. 269-315 (1994).

The term "diabodies" refers to small antibody fragments with two antigen binding sites, those fragments comprising a heavy chain variable domain (VH) connected to a light chain variable domain (VL) in the same polypeptide chain (VH-VL). By means of using a linker which is too short to allow pairing between the two domains in the same chain, the domains are forced to pair with the complementary domains of another chain and create two antigen binding sites. Diabodies are described in further detail in, for example, documents EP 404,097; WO 93/11161; and Hollinger et al., Proc. Natl. Acad. Sci. USA, 90: 6444-6448 (1993).

The term "nanobodies" designates small sized entities (15 kDa) formed solely by the antigen binding region of the heavy chain (VH fragment) of immunoglobulins. Said nanobodies are mainly produced after immunizing animals of the Camelidae family, such as camels, llamas and dromedaries, mainly llamas; and also of the shark family, which have the particularity of having antibodies which naturally lack the light chain and recognize the antigen by the heavy chain variable domain. Nevertheless, the nanobodies derived from these sources require a humanization process for their therapeutic application. Another potential source for obtaining nanobodies is from antibodies derived from different human samples by separating the VH and VL domains of the variable region. Nanobodies present advantages such as a production cost reduction with respect to whole antibodies, stability and the reduction of immunogenicity.

The term "antigen-binding region" of an antibody also includes a synthetic or a genetically engineered polypeptide that binds to a specific antigen, such as polypeptides consisting of the light chain variable region, "Fv" fragments consisting of the variable regions of the heavy and light chains, recombinant single chain polypeptide molecules in which light and heavy variable regions are connected by a peptide linker ("scFv proteins") and minimal recognition units consisting of the amino acid residues that mimic the hypervariable region.

### E. Method for identifying controller patients and for vaccine trials

The inventors have demonstrated that the presence of responses to a subset of highly conserved sequence elements (CE) and the maintenance of high avidity responses with broad variant recognition ability are hallmarks of a controlled HIV-1 infection.

Thus, in another aspect, the invention relates to a method for the identification of a HIV controller patient which comprises testing the capability of a sample containing T-cells isolated from said subject to mount a cytotoxic T cell response against at least one 10mer peptide derived from a conserved element within a COT sequence of an HIV protein. If compared to a reference sample the patient is capable of mounting a higher CTL response against at least one 10mer peptide, the subject is considered an HIV controller.

The term "HIV controller", as used herein, refers to an HIV infected subject who is able to maintain their virus at undetectable or low (<10,000 copies/ml) levels for many years in absence of treatment.

As used herein a "HIV controller" refers to a subject infected with HIV that exhibits a decrease in HIV viral load after the individual is infected with HIV and maintains the decreased HIV viral load over time. A "controller" also refers to an HIV-infected subject who remains asymptomatic with normal CD4 positive T-cell counts and low or undetectable plasma viral loads despite having never been treated with antiretroviral medications. HIV infected individuals that are controllers are capable of maintaining their viral load at a very low levels, for example plasma HIV RNA levels < 10,000 copies/ml and, preferably, less than 2000 copies/mL in the absence of antiretroviral therapy, measured three times over a period spanning at least 12 months. *See* Goudsmit J, et al., AIDS 2002; 16:791-793.

Features of controllers as defined by the HIV Controller Consortium are:
- Maintain HIV RNA levels below 2000 copies/mL
- No antiretroviral therapy for 1 year or longer
- Episodes of viremia are acceptable as long as they represent the minority of all available determinations

"Subject" means any animal or artificially modified animal. Animals include, but are not limited to, humans, non-human primates, cows, horses, sheep, goats, pigs, dogs, cats, rabbits, ferrets, rodents such as mice, rats and guinea pigs, and birds and fowl, such as chickens and turkeys. Artificially modified animals include, but are not limited to, transgenic animals or SCID mice with human immune systems. In the preferred embodiment, the subject is a human.

The terms "center-of-tree", "cytotoxic T cell response", "peptide", "conserved element" and "HIV protein" have been defined in detail above and are used in the same manner in relation to the method for identifying controller HIV patients according to the present invention.

The term "identification an HIV controller", as used herein refers to the determination of the likelihood that the patient will be able to maintain their virus at undetectable or low (< 10,000 copies/ml) levels for many years in the absence of treatment. As will be understood by those skilled in the art, the identification of controller individuals, although preferred to be, need not be correct for 100% of the subjects to be diagnosed or evaluated. The term, however, requires that a statistically significant portion of subjects can be identified as having an increased probability of being a controller. Whether a subject falls within the statistically significant group can be determined without further ado by the person skilled in the art using various well known statistic evaluation tools such as, for example, the determination of confidence intervals, p-value determination, Student's t-test and Mann-Whitney test. *See* Dowdy S, Wearden S, "Statistics for Research" (John Wiley & Sons, New York, NY, USA, 1983). Preferred confidence intervals are at least 50%, at least 60%, at least 70%, at least 80%, at least 90%, or at least 95%. The p-values are, preferably, 0.2, 0.1 or 0.05.

The method comprises a first step wherein a sample containing T-cells isolated from said patient to mount a cytotoxic T cell response against a peptide derived from a conserved element within a COT sequence of an HIV protein. The term "sample containing T cells" includes, without limitation, peripheral blood mononuclear cells, bone marrow, thymus, tissue biopsy, tumor, lymph node tissue, gut associated lymphoid tissue, mucosal-associated lymph node tissue, spleen tissue, or any other lymphoid tissue. T cells can be isolated as peripheral blood mononuclear cells (PBMC) from a blood sample obtained from the peripheral blood of a subject. PBMCs are prepared from peripheral blood by centrifugation on a gradient of Ficoll and Hypaque (metrizamide). *See* Walker M, et al., J. Clin. Invest., 2003; 112:1437-1443. Alternatively, T cells may be isolated from leukocytes derived from blood using an apheresis or leukopheresis procedure. Leukocytes derived from leukopheresis filters can be enriched for lymphocytes using density gradient centrifugation, as for example, through a Ficoll-metrizamide gradient.

Then, the samples containing T cells are placed in contact with one or more 10mer peptides derived from a conserved element within a COT region and their ability to mount a cytotoxic cell response is determined. The expression "mounting a cytotoxic T cell response", as used herein, refers to the ability of the T cell to proliferate as a result of the stimulation of the T cell receptor by one or more of the 10mer peptides.

In a preferred embodiment, the COT sequence is encoded by the *gag* gene. In a still more preferred embodiment, the protein encoded by the *gag* gene is p24.

In a preferred embodiment, the conserved element within the p24 COT sequence is selected from the group consisting of ISPRTLNAWVKV (SEQ ID NO:1), LSPRTLNAWVKV (SEQ ID NO:2), VIPMFSALSEGATPDQDLN (SEQ ID NO:3), VIPMFTALSEGATPDQDLN (SEQ ID NO:4), VGGHQAAMQMLKDTINEEAAEWDR (SEQ ID NO:5), VGGHQAAMQMLKETINEEAAEWDR (SEQ ID NO:6), PRGSDIAGTTSTLQEQIGW (SEQ ID NO:7), PRGSDIAGTTSTLQEQIAW (SEQ ID NO:8), KRWIILGLNKIVRMYSPVSI (SEQ ID NO:9), KRWIILGLNKIVRMYSPTSI (SEQ ID NO:10), YVDRFFKTLRAEQA (SEQ ID NO:11), YVDRFYKTLRAEQA(SEQ ID NO:12), LEEMMTACQGVGGPSHK (SEQ ID NO:13) and LEEMMTACQGVGGPGHK (SEQ ID NO:14).

In another preferred embodiment, the plurality of 10mer peptides contain one or more peptides selected from the group consisting of SPRTLNAWV (SEQ ID NO:15), ISPRTLNAW (SEQ ID NO:16), LSPRTLNAW (SEQ ID NO:17), EVIPMFSAL (SEQ ID NO:18), EVIPMFTAL (SEQ ID NO:19), VIPMFTAL (SEQ ID NO:20), SEGATPDQDL (SEQ ID NO:21), GHQAAMQML (SEQ ID NO:22), KDTINEEAA (SEQ ID NO:23), KETINEEAA (SEQ ID NO:24), DTINEEAAEW (SEQ ID NO:25), TSTLQEQIAW (SEQ ID NO:26), KRWIILGLNK (SEQ ID NO:27), GLNKIVRMY (SEQ ID NO:28), VRMYSPVSI (SEQ ID NO:29), VRMYSPTSI (SEQ ID NO:30), RMYSPVSI (SEQ ID NO:31), RMYSPTSI (SEQ ID NO:32), YVDRFFKTL (SEQ ID NO:33), YVDRFYKTL (SEQ ID NO:34), DRFFKTLRA (SEQ ID NO:35), and DRFYKTLRA (SEQ ID NO:36).

Once the cytotoxic T-cell response mounted by the T cell population has been determined, the subject is then classified as controller or not controller depending on whether the response is above the response observed in a reference sample.

The expression "reference sample", as used herein, refers to a sample which has been either not contacted with a peptide, or to a sample which has been contacted with a peptide which is incapable of triggering a cytotoxic cell response in the sample containing the T cells. Suitable peptides for use in the reference sample include any of the peptides shown above wherein the peptide sequence has been shuffled (i.e. the sequence of amino acids has been randomly altered maintaining the overall composition).

In a preferred embodiment, the determination of the CTL response is measured as functional avidity.

The term "functional avidity", as used herein, refers to the sum total of the strength of binding of two molecules to one another at multiple sites. Avidity is distinct from affinity, the latter of which refers to the binding strength between an antibody and a simple hapten or antigen determinant. In the case of CTL responses, avidity is typically given as the negative log of the peptide concentration that resulted in 50 percent maximal target lysis and is typically expressed in nM. *See* Derby M, et al., J. Immunol. 2001; 166:1690-1697.

The functional avidity of responses was determined by performing serial 10-fold limiting peptide dilutions ranging from 100µg/ml to 10pg/ml; in duplicates whenever enough PBMCs were available. Half-maximal stimulatory antigen doses (SD50%) were calculated as the peptide concentration needed to achieve a half-maximal number of spots in the ELISpot assay calculated by a sigmoidal dose response curve fit using GraphPad Prism4. Based on cell availability from the different subjects, 219 individual responses in the controllers group and 255 individual responses in the non-controllers group (representing 68% and 80% of all responses in the controllers and non-controllers groups, respectively) were titrated for the functional avidity analyses.

### F. Method for the identification of immunogenic peptides

In another aspect, the invention relates to a method for the identification of immunogenic peptides within a family of variant polypeptides which comprises the steps of:
(i) obtaining the center-of-tree sequence of the family of variant polypeptides,
(ii) identifying conserved sequence elements within the center-of-tree sequence,
(iii) producing peptides, the sequence of which is comprised within said conserved sequence elements, and
(iv) testing the peptides obtained in step for their immunogenicity.

In a first step, the method for the identification of immunogenic peptides according to the invention comprises obtaining the center-of-tree sequence of the family of variant polypeptides.

Typically, the family of variant polypeptides are viral antigens, such as retroviral antigens from the human immunodeficiency virus (HIV) including gene products of the *gag, pol, env* and *nef* genes, and other HIV components; hepatitis viral antigens, such as the S, M, and L proteins of hepatitis B virus, the pre-S antigen of hepatitis B virus, and other hepatitis (e.g. hepatitis A, B, and C, viral components such as hepatitis C viral RNA); influenza viral antigens, such as hemagglutinin and neuraminidase and other influenza viral components; measles viral antigens, such as the measles virus fusion protein and other measles virus components; rubella viral antigens, such as proteins El and E2 and other rubella virus components; rotaviral antigens, such as VP7sc and other rotaviral components; cytomegaloviral antigens, such as envelope glycoprotein B and other cytomegaloviral antigen components; respiratory syncytial viral antigens, such as the RSV fusion protein, the M2 protein and other respiratory syncytial viral antigen components; herpes simplex viral antigens, such as immediate early proteins, glycoprotein D, and other herpes simplex viral antigen components; varicella zoster viral antigens, such as gpI, gpII, and other varicella zoster viral antigen components; Japanese encephalitis viral antigens, such as proteins E, M-E, M-E-NSl, NSl, NS1-NS2A, 80 percent E, and other Japanese encephalitis viral antigen components; rabies viral antigens, such as rabies glycoprotein, rabies nucleoprotein and other rabies viral antigen components. *See* Fields B, Knipe D, Eds., "Fundamental Virology", 2nd Ed. (Raven Press, New York, NY, USA, 1991) for additional examples of viral antigens. Antigenic targets that may be delivered using the rAb -DC/DC -antigen vaccines of the present invention include genes encoding antigens such as viral antigens, bacterial antigens, fungal antigens or parasitic antigens. Viruses include picornavirus, coronavirus, togavirus, flavirvirus, rhabdovirus, paramyxovirus, orthomyxovirus, bunyavirus, arenavirus, reovirus, retrovirus, papilomavirus, parvovirus, herpesvirus, poxvirus, hepadnavirus, and spongiform virus. Other viral targets include influenza, herpes simplex virus 1 and 2, measles, dengue, smallpox, polio or HIV. Pathogens include trypanosomes, tapeworms, roundworms, helminthes or malaria. Tumor markers, such as fetal antigen or prostate specific antigen, may be targeted in this manner. Other examples include HIV Env proteins and hepatitis B surface antigen.

The determination of the centre-of-tree sequences from a family of variant sequences is usually carried out as described above. *See* Nickle D, et al., Science 2002; 296(5577):2354-2360; Rolland, 2007, supra; and Mullins, 2005, *supra.*

In a preferred embodiment, the sequences used for obtaining the COT sequence are of viral origin. In a still more preferred embodiment, the related peptides of viral origin are derived from HIV. In yet another embodiment, the sequences derived from HIV derive from a polypeptide encoded by the gag gene. In another preferred embodiment, the polypeptide encoded by the gag gene is p24.

In a second step, the method for the identification of immunogenic peptides within a family of variant polypeptides involves the identification of conserved sequence elements within the center-of-tree sequence. Conserved elements are those regions of the sequence that are substantially constant among all related variants when the variant sequences are compared. The identification of the conserved elements can be carried out as described above using any preferred pairwise or multiple sequence alignment algorithm.

In a third step, the method for the identification of immunogenic peptides within a family of variant polypeptides involves producing peptides, the sequence of which is comprised within said conserved sequence elements. Methods for obtaining and producing the peptides of the present invention are not particularly limited. Chemically synthesized peptides or recombinant peptides produced by gene recombination techniques are available. In a preferred embodiment, the peptides have a length of 10 amino acids or less.

In a fourth step, the method for the identification of immunogenic peptides within a family of variant polypeptide comprises testing the peptides obtained in the previous step for their immunogenicity. The step can be carried out essentially as described above based on the ability of the peptides to induce a biological response in a sample containing T cells such as proliferation assays, lymphokine secretion assays, direct cytotoxicity assays, and limiting dilution assays.

The invention is described hereinafter by way of the following examples that are merely illustrative and not limitative of the scope of the invention.

### EXAMPLES

### MATERIALS AND METHODS

### A. Study subjects

Chronically HIV-1 infected individuals were recruited from the HIV-1 Unit in Hospital Germans Trias i Pujol, Badalona, Spain. They fulfilled the following inclusion criteria: sustained viral load <10,000 RNA copies and CD4 cell counts >350 cells/mm³ for the controllers group (n=25) and viral load >50,000 RNA copies/ml and CD4 cell counts <350 cells/mm³ for the non-controllers (NC) group (n=25). Individuals with primary HIV-1 infection and subjects on antiretroviral treatment were excluded from the study. Furthermore, all patients were HLA typed at high resolution using SSP-PCR at the immunology department of the Hospital Germans Trias i Pujol. Individuals expressing any of the previously described protective alleles (HLA-B27, -B57 or -B58) were excluded from the analysis. *See* Mothe B, et al., Dis. Markers 2009; 27:105-120. Informed consent was obtained from all participants and the study was approved by the institutional review board of the Hospital Germans Trias i Pujol. Individuals in the NC group were slightly older than in the C group (p=0.04) but did not differ significantly with regards to time since diagnosis of HIV-1 infection. Likely route of HIV-1 acquisition and gender distribution was not significantly different in both groups either. The median HIV-1-1 RNA levels were 200,000 copies/ml (52,000-1,200,000) in NC. The median CD4 cell count was 642 cells/mm³ (434-1114) and 98 cells/mm³ (11-361) for the C and NC, respectively. These and additional demographic details are included in Table 3.

**Table 3. Demographic and main clinical characteristics of the 25 controllers and 25 non-controllers tested^{a}.**

| | **C (n=25)** | **NC (n=25)** | **P value** |
|---|---|---|---|
| Age, years | 38 (26.2-55.7) | 44.5 (24.3-54.8) | 0.04 |
| Time since HIV-1 diagnosis (years) | 9.3 (3.5-26.3) | 15.9 (1.5-23.3) | 0.07 |
| Gender (Female/Male) | F 40%/M 60% | F 40%/M 60% | |

| HIV risk group | | | |
|---|---|---|---|
| Heterosexual*^{b}* | 6 (24%) | 10 (40%) | 0.36 |
| Men who have sex with men*^{b}* | 8 (32%) | 4 (16%) | 0.32 |
| Injecting drug users*^{b}* | 7 (28%) | 9 (36%) | 0.76 |
| Other*^{b}* | 4 (16%) | 2 (8%) | 0.66 |
| Last CD4+ T cell counts (cells/mm³) | 642 (434-1114) | 98(11-361) | <0.001 |
| % CD4 cells | 32(16-50) | 9 (1-27) | <0.001 |
| Last HIV-1 RNA levels (copies/ml) | 810 (UD*^{C}*-10,000) | 200,000 (52,000-1,200,000) | <0.001 |

| HLA alleles representation | | | |
|---|---|---|---|
| HLA-A (n=24 alleles) | 20 alleles | 15 alleles | |
| HLA-B (n=34 alleles) | 27 alleles | 17 alleles | |
| HLA-C (n=20 alleles) | 17 alleles | 15 alleles | |

| | | | |
|---|---|---|---|
| *^{a}*Data are expressed as median (min-max range), *^{b}*n, (%), *^{c}*UD: undetectable viremia (<49 copies/ml) | | | |

### B. Synthetic peptides set

An overlapping peptide set of 223 peptides of 10 amino acids in length (overlapping by 9 residues) spanning the entire group M Center-of-Tree (COT-M) Gag p24 sequence was synthesized at the peptide synthesis facility of the Massachusetts General Hospital using 9-Fluorenylmethyloxycarbonyl (Fmoc)- chemistry. An additional eighty-eight 10-mer peptides were generated to cover the most frequently occurring variants in the 7 CE regions (1 variant per CE). In order to determine the relative immunodominance of Gag specific responses, the analysis also included a previously described comprehensive overlapping peptide (410 18mer OLP) set spanning the entire viral proteome. *See* Frahm N, et al., Aids 2008; 22:447-456. The OLP sequence was based on the consensus-B sequence of 2001 available at the HIV immunology database (http://www.hiv.lanl.gov/content/sequence/HIV/mainpage.html, September 2011). Peptides were generally 18mers varying from 15-20 amino acids in length and overlapping by 10 amino acids, designed using the PeptGen algorithm at the Los Alamos HIV database (http://www.hiv.lanl.gov/content/sequence/PEPTGEN/peptgen.html, September 2011).

### C. ELISpot assay

PBMCs were separated from whole blood within 4h of venopuncture and used directly for the IFN-γ ELISpot screening. Each COT-M Gag p24 overlapping peptide and the 88 variant peptides were added at a final concentration of 14µg/ml. For all assays, between 75,000 - 100,000 PBMC per well were added in 140ul of R10 96-well polyvinylidene plates (Millipore, Bedford, MA, USA). The IFN-γ Mabtech kit was used for detection of IFN-γ secretion following manufacturer instructions. In parallel, CTL responses to the clade B full proteome were assessed using the 18mer peptide set in a previously described matrix outline, followed by deconvolution of reactive pools and reconfirmation of each response at a single peptide level on the following day. *See* Frahm N, et al., J. Virol. 2004; 78:2187-2200. The number of spots was counted using a "CTL ELISpot Reader Unit" and the magnitude of responses was expressed as spot forming cells (SFC) per million input cells. The threshold for positive responses was defined as at least 5 spots per well and responses exceeding "mean number of spots in negative control wells plus 3 standard deviations of the negative control wells" and "three times the mean of negative control wells", whichever was higher. As a conservative approach and not to overestimate the breadth of responses, positive responses to 3 consecutive 10mers in the COT-M Gag p24 peptide set were counted as 1 response. Similarly, reactivity to 2 consecutive 18mer peptides was counted as 1 response when testing with the full proteome 18mer peptide set. The highest magnitude of the sequential responses was taken as the magnitude for each response. Responses reacting with flanking 10mer peptides that only partly overlapped with the CE regions were considered as targeting the actual CE when the 10mer overlapped with at least 8 amino acids of the CE.

### D. Functional avidity

The functional avidity of responses was determined by performing serial 10-fold limiting peptide dilutions ranging from 100µg/ml to 10pg/ml; in duplicates whenever enough PBMCs were available. Half-maximal stimulatory antigen doses (SD50%) were calculated as the peptide concentration needed to achieve a half-maximal number of spots in the ELISpot assay calculated by a sigmoidal dose response curve fit using GraphPad Prism4 (GraphPad Software Inc., San Diego, CA, USA). Based on cell availability, 219 individual responses in the controllers group and 255 individual responses in the non-controllers group -representing 68% and 80% of all responses in the controllers and non-controllers groups, respectively, were titrated for the functional avidity analyses.

### E. Gag p24 sequencing

Viral RNA was extracted from 1 millilitre of plasma spun at 9000g for 1.5 hour (QIAamp Viral RNA Kit™, QIAGEN, Valencia, CA, USA). From the pelleted RNA, the whole *gag* region was reverse-transcribed and amplified in a One-Step reaction (SuperScript® III One-Step RT-PCR System with Platinum® Taq High Fidelity, Invitrogen, Carlsbad, CA, USA) under the following conditions: 30 min at 52°C for the reverse transcription step; 2 min at 94°C; followed by 35 cycles at 94°C during 30 sec, 58°C during 30 sec and 68°C during 2 min; followed by a final extension step at 68°C during 5 min. Primers used for the RT-PCR were: Gag U761 (HXB2: 761→778) 5'-TTT GAC TAG CGG AGG CTA G-3' (SEQ ID NO:37) and Gag D2397 (HXB2: 2397→2376) 5'-CCC CTA TCA TTT TTG GTT TCC A-3' (SEQ ID NO:38). One microliter of the RT-PCR product was subsequently used as a template for a second, nested round of PCR (Platinum® Taq DNA Polymerase High Fidelity, Invitrogen, Carlsbad, CA, USA), using primers p24 U1070 (HXB2: 1070→1088) 5'-TAA AAG ACA CCA AGG AAG CT-3' (SEQ ID NO:39) and p24 D2063 (HXB2: 2063→2044) 5'-TCT TTC ATT TGG TGT CCT TC-3' (SEQ ID NO:40). PCR cycling conditions were: 2 min at 94°C; followed by 35 cycles at 94°C during 30 sec, 54°C during 30 sec and 68°C during 2 min; followed by a final extension step at 68°C during 5 min. The final PCR products were column-purified (QIAquick PCR Purification Kit, QIAGEN, Valencia, CA, USA) and sequenced bidirectionally. Sequences were assembled using Sequencher® 4.10.1 (Gene Codes Corp., Ann Arbor, MI, USA). Assembled sequences were codon-aligned using the Hidden Markov Model implemented in the tool HIValign (http://www.hiv.lanl.gov/content/index, September 2011). Autologous *gag* p24 bulk sequences were obtained for 21 of the 25 HIV-1 non-controllers included in our study.

### F. Statistical analyses

All values are presented as median values unless otherwise stated. GraphPad Prism version 4.00 for Windows (GraphPad Software Inc., San Diego, CA, USA) was used to compare response rates in both groups and subgroup analyses. Mann-Whitney test and Wilcoxon matched paired test were used for unpaired and paired comparisons, respectively. Spearman rank correlation was used to assess association between CD4 cell counts and the fraction of detectable responses targeting individual viral proteins.

### Example 1

### HIV-1 controllers and non-controllers show different immune focus on Gag and Nef proteins

A number of cohort-based analyses, some including several hundred individuals, have analyzed the differential contribution of protein-specific T cell responses to the overall cellular immunity against HIV-1. The most consistent observation is that HIV-1 infected individuals with relative control of *in vivo* viral replication target predominantly epitopes located in HIV-1 Gag. On the other hand, subjects with elevated viral loads were consistently found to mount their broadest and strongest responses towards HIV-1 Nef. Using a 18mer overlapping peptide set, we assessed the distribution of HIV-1-specific T cell responses in 25 HIV-1 controller and in 25 non-controllers by IFN-γ ELISpot assays. *See* Frahm, 2004, *supra.* To limit the potential bias by the presence of highly immunodominant epitopes restricted by HLA-B27 and-B57/58, whose responses are known to be associated with virologic control, the 50 tested subjects included only individuals without these HLA alleles. Overall, the majority of responses were detected against OLP located in the HIV-1 Gag, Pol and Nef proteins, as seen in earlier studies *See* Frahm, 2004, *supra.* HIV-1 controllers showed an overall broader breadth (median of 19 responses, range 7-53) than non-controllers (12 responses, range 2-55, p=0.025), translating also into a greater total magnitude of the response (18,881 SFC/10⁶ input PBMC, range 2,690-44,720) compared to non-controllers (9,000 SFC/10⁶ input PBMC, range 850-114,630, p=0.0353). The magnitude of individual responses in controllers and non-controllers was, however, not significantly different (p=0.2). As expected from earlier studies in another clade B infected cohort of comparable size in Peru, the HIV-1 controller subjects directed 29% of their total HIV-1-specific responses towards OLP located in Gag (18% in non-controllers, p=0.0336) whereas non-controllers targeted Nef more frequently (17% of their total responses) than controllers (12%, p=0.0076, data not shown). *See* Zuñiga R, et al., J. Virol. 2006; 80:3122-3125. We also found a statistically significant direct association between the relative breadth of Gag-specific CTL responses and the CD4 T-cell counts of the 50 individuals analyzed (p=0.03, Spearman's rank test, data not shown). Also, we detected an inverse correlation between relative immunodominance of Gag and the viral loads in the 50 individuals tested (p=0.0039, data not shown). These data confirm the associations seen between immunodominant Gag responses and lower viral loads, both in clade B and C infections, even in a small cohort of individuals without any protective HLA alleles, and suggest that an increased breath of the Gag response may provide increasing additional benefit for viral control.

### Example 2

### Responses to Gag p24 and in particular the CE regions are more frequent in HIV-1 controllers than in non-controllers

Among Gag-specific T cell responses, those targeting the p24 subunit have been particularly implicated in mediating viral control. *See* Zuñiga, 2006, *supra.* Indeed, more controllers showed a response to at least one Gag p24 OLP (24/25 individuals, 96%) compared to HIV-1 non-controllers (20/25, 80%). In addition, breadth of the Gag p24 response was higher in the controllers group as well, with a median of 3 responses to p24 in controllers (range 0-12) compared to 1 response in non-controllers (range 0-9, p=0.04). These differences were also significant when focusing on the 18 OLP (18mers) that span the 7 CE in p24, with a median of 2 responses in controllers (range 0-6) compared to 1 response in non-controllers (range 0-6, p=0.03). This data, when considered together, confirm earlier reports on the relative benefits of Gag specific responses. However, it also demonstrates clearly that Gag-specific responses can be detected in HIV-1 non-controllers. Why these responses do not contribute more effectively to viral control remains unclear. It may be possible that HIV-1 non-controllers mount responses to Gag at the same level as HIV-1 controllers, but with functional properties that do not allow their detection with the same ease as in controllers. Among different factors, the functional avidity of these responses and thereby the need for sensitive peptide test sets, may differ between the two groups.

### Example 3

### Gag p24 specific T cell responses in controllers and non-controllers are massively increased when using 10mer peptides spanning CE regions and rest of p24

In order to increase sensitivity of detection of responses, all individuals were tested against a set of 223 peptides of 10 amino acids in length (overlapping by 9 residues) spanning the group M Center-of-Tree (COT-M) Gag p24 sequence. Eighty-nine of the 223 10mer peptides covered the 7 CE regions that were identified when designing the CE-based p24 immunogen sequence (40%). This sequence represents a cross-clade, HIV-1 group M DNA immunogen designed to focus immune responses on conserved protein elements (CE) that are considered essential to the function of the virus while precluding responses against immunodominant decoys. The inclusion criteria for the CE was high sequence conservation within the HIV-1 group M (>98%). *See* Roland, 2007, *supra.* Sequence diversity coverage was further increased by including the single second most frequent variants at one amino acid for each CE. Allowing for the inclusion of such frequent "toggled" amino acids increased the CE coverage of all group M sequences to >99%. *See* Llano, 2009, *supra.* The 7 CE segments, as well as the optimal epitopes, have been described. *See* Figure 1.

When the COT-M Gag p24 10mers set of peptides was used in parallel to the 18mer OLP set (30 peptides, overlapping by 10 residues), significantly more responses were identified with the 10mers both in the controllers (p=0.0002) and non-controllers group (p=0.0006). Of note, the increase in response rates was more pronounced in the non-controller group (3-fold) than in the controllers (2-fold). This indicates that the non-controllers had proportionally greater enhancement of response detection towards the tested peptide in comparison to the controller group. *See* Figures 2A and 2B. As a consequence, the increased breadth of responses in the non-controllers also abolished the statistically significant broader responses to p24 that was noted earlier for the controllers utilizing the 18mer peptides: 6 responses (range 4-11) against p24 in controllers and 3 responses (range 1-18; p=0.42) in the non-controllers utilizing 10mers. Similarly, the responses to 10mers were of comparable magnitudes, both in terms of total magnitude (4,250 SFC/10⁶ PBMC in controllers (range 1,545-7,900) compared to 2,600 SFC/10⁶PBMC in non-controllers (range 240-12,985; p= 0.6, Figure 2C), the average magnitude of responses per individual (614 SFC/10⁶PBMC in controllers (range 87-4127) compared to 657 SFC/10⁶PBMC in non-controllers (range 70-2414, p=0.91, Figure 2D) and when restricting the analyses to those responses to the same 10-mer peptides detected both in controllers and non-controllers (n=82 responses, p=0.16; data not shown). The same results emerged when comparing responses to the CE regions only, showing comparable breadth (median of 2) and magnitude by controllers and non-controllers, respectively. *See* Figures 2E and 2F. These results demonstrate that p24 specific responses in HIV-1 non-controllers are readily detected when using a sensitive 10mer peptide set and that they are unlikely to represent spurious, nonspecific reactivities.

### Example 4

### CE containing HLA-B14, -B27 and B57 restricted, protective CTL epitopes are predominantly targeted by HIV-1 controllers in the absence of individuals expressing these protective alleles

As HIV-1 controllers and non-controllers showed comparable total response rates to the 7 CE, we next asked whether any of the individual CE performed significantly differently between the two groups. Indeed, three CE were targeted by at least 40% more controllers than non-controllers (CE #4, #5 and #6, Figure 3A): 19 controllers and 13 non-controllers had a detectable response to CE-4 (p=0.14). Similarly, CE #5 was targeted by 17 controllers and 10 non-controllers (p=0.08) whereas 3 times more controllers (n=15) made a response against CE #6 than non-controllers (n=5, p=0.009). Furthermore, and in line with the observation that an increased breadth of responses to Gag was associated with superior viral control, detection of responses to the combination of these three CE ("CE 4+5+6") was significantly higher in controllers (median of 3 responses to CE 4+5+6) compared to non-controllers (median 1 response, p=0.0006; Figure 3B). In addition, despite an overall high variability, the total magnitude of the responses to CE 4+5+6 gave a weak, though significant, correlation with HIV-1 viral load (r=-0.5, p=0.0002 by Spearman's rank, Figure 3C), suggesting that stronger responses to these three regions mediates better control of viral replication. Of note, the 3 regions included the well-studied HLA-B57 restricted TW10 epitope (in CE #4), the HLA-B27 restricted KK10 epitope (in CE #5), and the HLA-B14 restricted DA9 epitope (in CE #6), all of which have been previously associated with containment of HIV-1 replication in HIV-1 controllers. *See* Brumme Z, et al., J. Virol. 2008; 82:9216-9927; Goulder P, et al., J. Exp. Med. 2001; 193:181-194; Goulder P, et al., Nature 2001; 412:334-338; and Pereyra F, et al., AIDS Vaccine Meeting, Cape Town, South Africa, 2008. Remarkably in our study, HLA-B57+ and -B27+ individuals were excluded and just 1 controller and 1 non-controller expressed the HLA-B14 allele among the responders to the peptides covering the B14 DA9 epitope. This indicates that mounting responses to these regions is particularly effective in HIV-1 controllers even if these protective epitopes are being presented on different HLA class I molecules than those described in original studies. *See* Frahm, 2007, *supra.* Supporting this widely promiscuous epitope presentation is the observation that 75% of responses against CE regions that contained a known optimal epitope were seen in individuals that did not express the described restricting HLA class I allele. In addition, 35% of the more than 300 individual responses against the CE segments were targeting peptides that did not contain known optimal HIV-1 CTL epitopes. These data suggests that the CE regions included in our CEvac design represented a very rich array of responses that are not being blocked from presentation in natural chronic infection and that are able to be recognized in a wide HLA class I context.

### Example 5

### HIV-1 controllers showed higher variant recognition

Although the CE-immunogen approach is based on the identification of the most conserved segments of the HIV-1 genome, the inclusion of single second most frequent variants can further increase coverage of global group M sequence diversity. To incorporate these variants in the *ex-vivo* immune analyses, the 223 10mer peptides included in the above screenings were complemented by 88 additional overlapping 10mer variant peptides; 42 of which were covering sequence variants located in CE regions. As for the COT-M sequence peptides, the median number of responses to these p24 variants was greater in controllers (median of 4 responses; range 2-9) than in HIV-1 non-controllers (median 2 responses, range 0-16) and reached statistical significance despite the smaller number of tested peptides (p=0.02). *See* Figure 4A. The increase was more statistically significant when limiting the analyses to variants located in CE regions only (median of 2 responses in controllers *versus* 1 response in non-controllers, p=0.01). As seen for the 223 peptides set, the average magnitude of the variant-specific responses was comparable between controllers (median of 742 SFC/10⁶PBMC, range 90-3,073) and non-controllers (median 473 SFC/10⁶PBMC, range 60-2,707; p=0.56). *See* Figure 4B.

The variant recognition data was also analyzed in comparison to the reactivity to the "wild type" COT-M sequence. This allowed for the identification of cases in which responses were only detected by using the variant peptide and the assessment of the level of cross-reactivity of responses (i.e. responses that reacted with both the COT-M and the variant peptides). Of the 50 subjects tested, 23 controllers and 17 non-controllers had detectable responses to COT-M peptides for which a variant peptide was tested. Among controllers, a median of 50% of variant peptides were reactive when the COT-M sequence elicited a response. In HIV-1 non-controllers, individuals reacted with a median of 31% to the tested variants of reactive COT-M peptides (p =0.2). *See* Figure 4C. While this did not reach statistical significance, controllers reacted with significantly more variant peptides for which the COT-M sequence did not elicit a response (median of 2 gained responses by inclusion of variants, range 0-7) than the non-controllers (median 1, range 0-5; p=0.03). *See* Figure 4D.

To rule out that the overall reduced variant response rates in the non-controllers was due to mismatches between the tested peptide sequences and the autologous viral sequence in the CE region, autologous Gag p24 bulk sequences were obtained for 21 of the 25 HIV-1 non-controllers. The analysis showed a 99% identity between the test set and the autologous viral sequences as only 24 amino acid polymorphisms were identified across all 7 CE segments for the 21 individuals analyzed. *See* Figure 5. This suggests that the relative absence of variant reactivity in HIV-1 non-controllers is caused by their impaired ability to mount or maintain cross-reactive T cell responses rather than to mismatches between tested peptide sequences and autologous viruses. Together, the data indicate that HIV-1 controllers can react with more epitope variants than non-controllers and together with data in Figure 2, show that this superior variant recognition was not simply due to the presence of responses of greater magnitudes in the controllers.

### Example 6

### Broadly cross-reactive responses in HIV-1 controllers are of higher functional avidity than in non-controllers and mediate better variant recognition than low-avidity responses

Based on cell availability, a total of 474 individual responses (219 responses in HIV-1 controllers and 255 responses in non-controllers) were titrated to define their functional avidity. As shown in Figure 6A, controllers showed responses of higher functional avidity (median 6,110 ng/ml, range 0.05-7.6x10⁷) compared to responses detected in non-controllers (median of 13,548 ng/ml, range 0.64-4x10⁹; p=0.01) The difference in functional avidity was even more pronounced when the analysis was limited to the 52 10mer-specific responses that were seen in both the controller and non-controller group (6,998 ng/ml *vs*. 46,637 ng/ml, respectively; p=0.01 Wilcoxon) *See* Figure 6B.

In order to test directly whether functional avidity was related to the ability to recognize peptide variants, titrated responses were grouped into high, intermediate and low avidity responses and their variant recognition potentials were compared. Indeed, responses with SD50% in the first quartile of all titrated responses (<1,401ng/ml) showed cross-reactivity with their variants in 67% of all cases, whereas fewer (48% and 33%) of responses of intermediate or low functional avidity were cross-reactive with their 10mer variants. *See* Figure 6C. In order to avoid bias, in the assay with individuals showing particularly broad responses, data was also analyzed by determining the fraction of cross-reactive responses per individual after separating the subjects' responses in either high, intermediate or low avidity activities -defined as mentioned before. In the majority of cases, responses of high functional avidity also reacted with the tested variants (median 75%), while intermediate and low avidity responses failed to recognize the peptide variants (median of 50% and 0% variant recognition, respectively; p=0.0018 ANOVA, data not shown). Altogether, the data demonstrate that high avidity responses were more prevalent in HIV-1 controllers and mediated superior variant recognition than responses of low functional avidity. The data also identify epitope variants that are reactive when the COT-M sequence did not elicit a response, providing additional rationale for inclusion of sequence variants in HIV-1 vaccine immunogen sequences.

## Claims

1. An isolated polynucleotide encoding a polypeptide comprising one or more peptides selected from the group consisting of SEQ ID NOs: 3, 5, 7, 10, 12, 14, and 15, wherein the polynucleotide does not encode an HIV gag protein.

2. The isolated polynucleotide of claim 1 encoding two or more peptides selected from the group.

3. The isolated polynucleotide of claim 1 encoding three or more peptides selected from the group.

4. The isolated polynucleotide of claim 1 encoding four or more peptides selected from the group.

5. The isolated polynucleotide of claim 1 encoding five or more peptides selected from the group.

6. The isolated polynucleotide of claim 1 encoding six or more peptides selected from the group.

7. The isolated polynucleotide of claim 1 encoding SEQ ID NOs: 3, 5, 7, 10, 12, 14, and 15.

8. A recombinant expression vector comprising the isolated polynucleotide according to claim 1.

9. An immunogenic composition comprising the isolated polynucleotide according to claim 1 and a pharmaceutically acceptable carrier.

10. An immunogenic composition comprising the vector according to claim 8 and a pharmaceutically acceptable carrier.

11. A recombinant polypeptide comprising of one or more peptides selected from the group consisting of SEQ ID NOs: 3, 5, 7, 10, 12, 14, and 15, wherein the recombinant polypeptide does not encode an HIV gag protein.

12. An immunogenic composition comprising the recombinant polypeptide of claim 11 and a pharmaceutically acceptable carrier.

13. An amount effective of the polynucleotide of any one of claims 1 to 7, of the immunogenic compositions of any of claims 9, 10 or 12 or of the recombinant polypeptide of claim 11 for use in the treatment of a disease resulting from HIV-infection in a subject.

14. An amount effective of the polynucleotide of any one of claims 1 to 7, of the immunogenic compositions of any of claims 9, 10 or 12 or of the recombinant polypeptide of claim 11 for use in the generation of a cytotoxic T lymphocyte response in a subject.


SEQUENCE LISTING

<110>  Laboratorios del Dr. Esteve S.A.
       Fundació Privada Institut de Recerca de la SIDA-Caixa
       Institució Catalana de Recerca i Estudis Avançats
       University of Washington

<120>  HIGHLY IMMUNOGENIC HIV P24 SEQUENCES

<130>  P6526PC00

<150>  US61/412060
<151>  2010-11-10

<160>  42

<170>  PatentIn version 3.5

<210>  1
<211>  12
<212>  PRT
<213>  Artificial

<220>
<223>  p24 conserved element

<400>  1

Ile Ser Pro Arg Thr Leu Asn Ala Trp Val Lys Val
1               5                   10


<210>  2
<211>  12
<212>  PRT
<213>  Artificial

<220>
<223>  p24 conserved element

<400>  2

Leu Ser Pro Arg Thr Leu Asn Ala Trp Val Lys Val
1               5                   10


<210>  3
<211>  19
<212>  PRT
<213>  Artificial

<220>
<223>  p24 conserved element

<400>  3

Val Ile Pro Met Phe Ser Ala Leu Ser Glu Gly Ala Thr Pro Asp Gln
1               5                   10                  15


Asp Leu Asn



<210>  4

<211>  19
<212>  PRT
<213>  Artificial

<220>
<223>  p24 conserved element

<400>  4

Val Ile Pro Met Phe Thr Ala Leu Ser Glu Gly Ala Thr Pro Asp Gln
1               5                   10                  15

Asp Leu Asn

<210>  5
<211>  24
<212>  PRT
<213>  Artificial

<220>
<223>  p24 conserved element

<400>  5

Val Gly Gly His Gln Ala Ala Met Gln Met Leu Lys Asp Thr Ile Asn
1               5                   10                  15

Glu Glu Ala Ala Glu Trp Asp Arg
            20

<210>  6
<211>  24
<212>  PRT
<213>  Artificial

<220>
<223>  p24 conserved element

<400>  6

Val Gly Gly His Gln Ala Ala Met Gln Met Leu Lys Glu Thr Ile Asn
1               5                   10                  15

Glu Glu Ala Ala Glu Trp Asp Arg
            20

<210>  7
<211>  19
<212>  PRT
<213>  Artificial

<220>
<223>  p24 conserved element

<400>  7

Pro Arg Gly Ser Asp Ile Ala Gly Thr Thr Ser Thr Leu Gln Glu Gln
1               5                   10                  15

Ile Gly Trp

<210>  8
<211>  19
<212>  PRT
<213>  Artificial

<220>
<223>  p24 conserved element

<400>  8

Pro Arg Gly Ser Asp Ile Ala Gly Thr Thr Ser Thr Leu Gln Glu Gln
1               5                   10                  15

Ile Ala Trp

<210>  9
<211>  20
<212>  PRT
<213>  Artificial

<220>
<223>  p24 conserved element

<400>  9

Lys Arg Trp Ile Ile Leu Gly Leu Asn Lys Ile Val Arg Met Tyr Ser
1               5                   10                  15

Pro Val Ser Ile
            20

<210>  10
<211>  20
<212>  PRT
<213>  Artificial

<220>
<223>  p24 conserved element

<400>  10

Lys Arg Trp Ile Ile Leu Gly Leu Asn Lys Ile Val Arg Met Tyr Ser
1               5                   10                  15

Pro Thr Ser Ile
            20

<210>  11
<211>  14
<212>  PRT
<213>  Artificial

<220>
<223>  p24 conserved element

<400>  11

Tyr Val Asp Arg Phe Phe Lys Thr Leu Arg Ala Glu Gln Ala
1               5                   10


<210>  12
<211>  14
<212>  PRT
<213>  Artificial

<220>
<223>  p24 conserved element

<400>  12

Tyr Val Asp Arg Phe Tyr Lys Thr Leu Arg Ala Glu Gln Ala
1               5                   10


<210>  13
<211>  17
<212>  PRT
<213>  Artificial

<220>
<223>  p24 conserved element

<400>  13

Leu Glu Glu Met Met Thr Ala Cys Gln Gly Val Gly Gly Pro Ser His
1               5                   10                  15


Lys




<210>  14
<211>  17
<212>  PRT
<213>  Artificial

<220>
<223>  p24 conserved element

<400>  14

Leu Glu Glu Met Met Thr Ala Cys Gln Gly Val Gly Gly Pro Gly His
1               5                   10                  15


Lys




<210>  15
<211>  9
<212>  PRT
<213>  Artificial

<220>

<223>  p24 peptide

<400>  15

Ser Pro Arg Thr Leu Asn Ala Trp Val
1               5

<210>  16
<211>  9
<212>  PRT
<213>  Artificial

<220>
<223>  p24 peptide

<400>  16

Ile Ser Pro Arg Thr Leu Asn Ala Trp
1               5

<210>  17
<211>  9
<212>  PRT
<213>  Artificial

<220>
<223>  p24 peptide

<400>  17

Leu Ser Pro Arg Thr Leu Asn Ala Trp
1               5

<210>  18
<211>  9
<212>  PRT
<213>  Artificial

<220>
<223>  p24 peptide

<400>  18

Glu Val Ile Pro Met Phe Ser Ala Leu
1               5

<210>  19
<211>  9
<212>  PRT
<213>  Artificial

<220>
<223>  p24 peptide

<400>  19

Glu Val Ile Pro Met Phe Thr Ala Leu
1               5

<210>  20

<211>  8
<212>  PRT
<213>  Artificial

<220>
<223>  p24 peptide

<400>  20

Val Ile Pro Met Phe Thr Ala Leu
1               5


<210>  21
<211>  10
<212>  PRT
<213>  Artificial

<220>
<223>  p24 peptide

<400>  21

Ser Glu Gly Ala Thr Pro Asp Gln Asp Leu
1               5                   10


<210>  22
<211>  9
<212>  PRT
<213>  Artificial

<220>
<223>  p24 peptide

<400>  22

Gly His Gln Ala Ala Met Gln Met Leu
1               5


<210>  23
<211>  9
<212>  PRT
<213>  Artificial

<220>
<223>  p24 peptide

<400>  23

Lys Asp Thr Ile Asn Glu Glu Ala Ala
1               5


<210>  24
<211>  9
<212>  PRT
<213>  Artificial

<220>
<223>  p24 peptide

<400>  24

Lys Glu Thr Ile Asn Glu Glu Ala Ala
1               5

<210>  25
<211>  10
<212>  PRT
<213>  Artificial

<220>
<223>  p24 peptide

<400>  25

Asp Thr Ile Asn Glu Glu Ala Ala Glu Trp
1               5                   10

<210>  26
<211>  10
<212>  PRT
<213>  Artificial

<220>
<223>  p24 peptide

<400>  26

Thr Ser Thr Leu Gln Glu Gln Ile Ala Trp
1               5                   10

<210>  27
<211>  10
<212>  PRT
<213>  Artificial

<220>
<223>  p24 peptide

<400>  27

Lys Arg Trp Ile Ile Leu Gly Leu Asn Lys
1               5                   10

<210>  28
<211>  9
<212>  PRT
<213>  Artificial

<220>
<223>  p24 peptide

<400>  28

Gly Leu Asn Lys Ile Val Arg Met Tyr
1               5

<210>  29
<211>  9
<212>  PRT
<213>  Artificial

<220>
<223>  p24 peptide

<400>  29

Val Arg Met Tyr Ser Pro Val Ser Ile
1               5


<210>  30
<211>  9
<212>  PRT
<213>  Artificial

<220>
<223>  p24 peptide

<400>  30

Val Arg Met Tyr Ser Pro Thr Ser Ile
1               5


<210>  31
<211>  8
<212>  PRT
<213>  Artificial

<220>
<223>  p24 peptide

<400>  31

Arg Met Tyr Ser Pro Val Ser Ile
1               5


<210>  32
<211>  8
<212>  PRT
<213>  Artificial

<220>
<223>  p24 peptide

<400>  32

Arg Met Tyr Ser Pro Thr Ser Ile
1               5


<210>  33
<211>  9
<212>  PRT
<213>  Artificial

<220>
<223>  p24 peptide

<400>  33

Tyr Val Asp Arg Phe Phe Lys Thr Leu
1               5

<210>  34
<211>  9
<212>  PRT
<213>  Artificial

<220>
<223>  p24 peptide

<400>  34

Tyr Val Asp Arg Phe Tyr Lys Thr Leu
1               5


<210>  35
<211>  9
<212>  PRT
<213>  Artificial

<220>
<223>  p24 peptide

<400>  35

Asp Arg Phe Phe Lys Thr Leu Arg Ala
1               5


<210>  36
<211>  9
<212>  PRT
<213>  Artificial

<220>
<223>  p24 peptide

<400>  36

Asp Arg Phe Tyr Lys Thr Leu Arg Ala
1               5


<210>  37
<211>  19
<212>  DNA
<213>  Artificial

<220>
<223>  Primer Gag U761

<400>  37
tttgactagc ggaggctag                                                    19


<210>  38
<211>  21
<212>  DNA
<213>  Artificial

<220>
<223>  Primer Gag D2397

<400>  38
   cccctatcat ttttggtttc c                                                  21

<210>  39
<211>  20
<212>  DNA
<213>  Artificial

<220>
<223>  Primer p24 U1070

<400>  39
taaaagacac caaggaagct                                                   20


<210>  40
<211>  20
<212>  DNA
<213>  Artificial

<220>
<223>  Primer p24 D2063

<400>  40
tctttcattt ggtgtccttc                                                   20


<210>  41
<211>  231
<212>  PRT
<213>  HIV-1

<400>  41

Pro Ile Val Gln Asn Leu Gln Gly Gln Met Val His Gln Ala Ile Ser
1               5                   10                  15

Pro Arg Thr Leu Asn Ala Trp Val Lys Val Val Glu Glu Lys Ala Phe
            20                  25                  30

Ser Pro Glu Val Ile Pro Met Phe Ser Ala Leu Ser Glu Gly Ala Thr
        35                  40                  45

Pro Gln Asp Leu Asn Thr Met Leu Asn Thr Val Gly Gly His Gln Ala
    50                  55                  60

Ala Met Gln Met Leu Lys Asp Thr Ile Asn Glu Glu Ala Ala Glu Trp
65                  70                  75                  80

Asp Arg Leu His Pro Val His Ala Gly Pro Ile Pro Pro Gly Gln Met
                85                  90                  95

Arg Glu Pro Arg Gly Ser Asp Ile Ala Gly Thr Thr Ser Thr Leu Gln
            100                 105                 110

Glu Gln Ile Gly Trp Met Thr Ser Asn Pro Pro Ile Pro Val Gly Glu
        115                 120                 125

Ile Tyr Lys Arg Trp Ile Ile Leu Gly Leu Asn Lys Ile Val Arg Met

130                 135                 140

Tyr Ser Pro Val Ser Ile Leu Asp Ile Arg Gln Gly Pro Lys Glu Pro
145                 150                 155                 160

Phe Arg Asp Tyr Val Asp Arg Phe Phe Lys Thr Leu Arg Ala Glu Gln
                165                 170                 175

Ala Thr Gln Asp Val Lys Asn Trp Met Thr Asp Thr Leu Leu Val Gln
            180                 185                 190

Asn Ala Asn Pro Asp Cys Lys Thr Ile Leu Lys Ala Leu Gly Pro Gly
        195                 200                 205

Ala Thr Leu Glu Glu Met Met Thr Ala Cys Gln Gly Val Gly Gly Pro
    210                 215                 220

Ser His Lys Ala Arg Val Leu
225                 230

<210>  42
<211>  231
<212>  PRT
<213>  HIV-1

<400>  42

Pro Ile Val Gln Asn Leu Gln Gly Gln Met Val His Gln Ala Leu Ser
1               5                   10                  15

Pro Arg Thr Leu Asn Ala Trp Val Lys Val Val Glu Glu Lys Ala Phe
            20                  25                  30

Ser Pro Glu Val Ile Pro Met Phe Thr Ala Leu Ser Glu Gly Ala Thr
        35                  40                  45

Pro Gln Asp Leu Asn Thr Met Leu Asn Thr Val Gly Gly His Gln Ala
    50                  55                  60

Ala Met Gln Met Leu Lys Glu Thr Ile Asn Glu Glu Ala Ala Glu Trp
65                  70                  75                  80

Asp Arg Leu His Pro Val His Ala Gly Pro Ile Pro Pro Gly Gln Met
                85                  90                  95

Arg Glu Pro Arg Gly Ser Asp Ile Ala Gly Thr Thr Ser Thr Leu Gln
            100                 105                 110

Glu Gln Ile Ala Trp Met Thr Ser Asn Pro Pro Ile Pro Val Gly Glu
        115                 120                 125

Ile Tyr Lys Arg Trp Ile Ile Leu Gly Leu Asn Lys Ile Val Arg Met
    130                 135                 140

Tyr Ser Pro Thr Ser Ile Leu Asp Ile Arg Gln Gly Pro Lys Glu Pro
145                 150                 155                 160

Phe Arg Asp Tyr Val Asp Arg Phe Tyr Lys Thr Leu Arg Ala Glu Gln
                165                 170                 175

Ala Thr Gln Asp Val Lys Asn Trp Met Thr Asp Thr Leu Leu Val Gln
            180                 185                 190

Asn Ala Asn Pro Asp Cys Lys Thr Ile Leu Lys Ala Leu Gly Pro Gly
        195                 200                 205

Ala Thr Leu Glu Glu Met Met Thr Ala Cys Gln Gly Val Gly Gly Pro
    210                 215                 220

Gly His Lys Ala Arg Val Leu
225                 230